# EUROPEAN PATENT APPLICATION

(11) **EP 3 100 738 A1**
(43) Date of publication of application: **07.12.2016**
(21) Application number: 16020175.2
(22) Date of filing: 09.05.2016
(51) Int. Cl.: A61K 35/28, A61K 47/00, A61K 31/728, A61P 19/02

(54) **METHOD AND PREPARATION FOR THE TREATMENT OF ARTICULAR CARTILAGE INJURY COMPRISING MESENCHYMAL STEM CELLS AND HYALURONIC ACID**

(30) Priority: 08.05.2015 HU 1500218
(71) Applicant: DeltaBio 2000 Kft, 6726 Szeged (HU); Magyar Tudományos Akadémia SzegediBiológiai Kutatoközpont, 6726 Szeged (HU)
(72) Inventor: HARACSKA, Lajos, H-6726 Szeged (HU); MONOSTORI, Éva, H-6771 Szeged (HU); KRISTON-PÁL, Éva, H-3704 Berente (HU); GERCSÓ, Andrásné, H-6723 Szeged (HU); ILLÉSNÉ KOVÁCS, Katalin, H-6710 Szeged (HU); UHER, Ferenc, H-1122 Budapest (HU)
(74) Representative: Svingor, Adam

(57) **Abstract**

The present invention relates to a composition and a therapeutic procedure for the treatment of articular cartilage injuries, preferably joint dysplasia and orthopaedic diseases (e.g. partial or complete ligament tear, patellar dislocation) in non-human, mammalian patients.

## Description

### FIELD OF THE INVENTION

The subject matter of the invention is a preparation and a therapeutic method for the treatment of articular cartilage injury, preferably arthrodegenerative orthopaedic diseases, articular orthopaedic diseases involving arthrosis and cartilage detachment, osteoarthritis, dysplasia, and articular traumatic diseases of non-human mammalian patients.

### STATE OF TECHNOLOGY

Treatment of articular cartilage damage, particularly the degenerative deformations of the cartilage, is one of the greatest challenges of our time, which remains to be solved both in veterinary and human medicine. We present the degenerative diseases of the articular cartilage through the example of the particularly significant cartilage damage arising during dysplasia.

Elbow dysplasia (ED) is a developmental disorder affecting a large number of dogs. ED occurs primarily in medium- and large-sized dog breeds, and often develops early, at around the age of 4-8 months. The disease is similar to the genetic detachment of the cartilage. Currently, generally used treatments involve surgical removal of the detached cartilage, anti-inflammatory and analgesic drugs, and cartilage-fortifying supplements.

It is known in general that mesenchymal stem cells (MSC) can be used to treat cartilage injuries.

International publication No. WO1997018299A1 relates to the differentiation of MSCs into chondrocytes by a chondroinductive agent.

International publication No. WO2005025493A2 describes an osteochondral construct and its engineering via the culturing of a first portion of MSCs in a chondrogenic medium to induce differentiation into chondrocytes and the culturing of a second portion of the cells in an osteogenic medium to induce differentiation into osteoblasts. Finally, the cells are loaded into a biocompatible scaffold.

Furthermore, international publication No. WO2014052912 encompasses the differentiation of fibro-blast-derived induced pluripotent stem cell aggregates into MSCs and then the differentiation of MSCs into chondrocytes. The aim of the procedure is the treatment of cartilage injuries.

Huselstein et al. [Huselstein et al. Bio-Medical Materials and Engineering 22 (2012) 69-80] also established that MSCs can be used in regenerative medicine for articular cartilage regeneration via autologous transplantation. The authors discuss the improvement of injured cartilaginous tissue via the transplantation of MSCs as an alternative to autologous chondrocyte transplantation. The authors present the possibilities for the employment of autologous chondrocytes and MSCs in medicine and summarize the advantages and disadvantages of these cell types regarding the formation of articular cartilage.

Vinatier et al. [Vinatier C. Curr Stem Cell Res Ther. Dec 2009; 4(4): 318-329.] express their opinion in their review that MSCs are suitable sources of cells for the regeneration of cartilage; they are easily accessible and can be cultured *in vitro*. They also note that MSCs can be isolated from several tissues such as bone marrow, adipose tissue, synovium, umbilical cord, and placenta. The authors describe that hyaluronic acid (HA), which is a component of the extracellular matrix (EMC) and thus of cartilaginous ECM, increases the synthesis of ECM by chondrocytes *in vitro* and *in vivo,* but they conclude that in its unmodified form HA is not suitable for cartilage repair treatment and requires crosslinking to enhance its biocompatibility. HA may also be combined with other matrices such as gelatine, chondroitin, and HA sulphate.

Ayhan et al. **[**Ayhan et al. World J Orthop 2014 July 18; 5(3): 351-361] concede that intraarticular hyaluronic acid injections can be effective for short term pain relief in patients with mild osteoarthritis, but they emphasize that the cost-effectiveness of the treatment is dubious.

Tan H et al. [Tan H et al. J Tissue Eng Regen Med. 2011 November ; 5(10): 790-797] mention that over the past period several methods have been employed for the preparation of injectable polysaccharide hydrogels for gelation, including ionic interactions, photopolymerization, and chemical crosslinking. The authors crosslink aminated and oxidized HA with the genipin compound. Employment without crosslinking is not raised.

Chung et al. [Chung et al. Stem Cell Research & Therapy 2014, 5:39] report in their article in 2014 the testing of composites of human umbilical cord blood-derived MSCs and four different hydrogel matrices for articular cartilage repair in a rat model. The authors published promising results of treatment with human MSCs mixed with hydrogels immediately prior to application and report that hyaluronic acid was the most appropriate among the hydrogels.

The use of HA is analysed in detail in international publication No. WO2013110056(A1**)** as well. It is established that HA-based scaffolds employed in practice exhibit little similarity to natural HA and are still not stable enough, representing the main obstacle to their use, and describe a stable crosslinked HA.

Guilak et al. [Guilak et al. Clin Orthop Relat Res (2010) 468:2530-2540)] describe that adipose tissue is an abundant source of multipotent progenitor cells. However, Jacobsen et al. [Jakobsen RB et al. Knee Surg Sports Traumatol Arthrosc. 2010 18(10):1407-16.] compared different cells for their chondrogenic ability in a HA scaffold and found that bone marrow-derived MSCs are more efficient than both the chondrocytes and the adipose tissue-derived MSCs. Though bone marrow-derived MSCs are more efficient in transforming into cartilage, MSCs isolated from adipose tissue are also capable of differentiating into cartilage. The advantage of adipose tissue-derived MSCs is their greater number and ease of accessibility as compared to bone marrow-derived MSCs.

Patrascu J. M. et al [Patrascu JM et al. J Biomed Mater Res B Appl Biomater. 2013 Oct;101(7):1310-20. doi: 10.1002/jbm.b.32944. Epub 2013 May 10.] found that *in vitro* chondrogenic differentiation and cartilage repair could be observed in polyglycolic acid-hyaluronan scaffolds loaded with bone marrow-derived MSCs in the rabbit model.

Mohand-Kaci et al. reported that an optimized HA-hydrogel composition is beneficial for the ability of stem cells to divide in culture [Mohand-Kaci et al. Eng Part C Methods. 2013 19(4):288-98.].

At the same time Gómez-Aristizábal, Alejandro et al. [Gómez-Aristizábal, Alejandro et al. PlosOne (2016) January 28, 1-18, e-pub] carried out a systematic study by adding HAs with MWs of 1.6 MDa (high molecular weight hyaluronic acid, hHA), 150 kDa or 7.5 kDa (HA) to MSCs alone or to MSC-immune cell cocultures to investigate their immunomodulatory effects in the treatment of osteoarthritis (OA). The authors concluded that hHA provides a less pro-inflammatory environment than HA; thus, they recommend the former for the treatment of OA.

Despite the advances reviewed above, Qi Y et al. [Qi Y et al. Mol Biol Rep. 2012 39(5):5683-9.] found in 2012 that the restoration of the injured articular cartilage in patients with osteoarthritis (OA) is still a challenge for both researchers and clinicians, and while trials have been performed using composites of scaffolds and MSCs, the intra-articular injection of MSCs without a scaffold would represent a more favourable alternative.

Hoogduijn MJ et al. [Hoogduijn MJ et al. Curr Opin Organ Transplant. 2014 Feb;19(1):41-6.] conclude in their review that MSC research in organ transplantation is currently moving from safety-feasibility studies to efficacy. Although the differences between MSCs from different tissue sources are subtle, they may affect their efficacy. The impact of culture conditions on MSCs may be even greater and can be exploited to induce the desired properties in MSCs prior to transplantation. MSCs are short-lived after infusion, and this time period is of great importance for the interaction between MSCs and the host cells. The authors state that the success of a future therapy lies in the appropriate choice of the source, the culturing, and the treatment of cells.

Though the differentiation ability of MSCs was demonstrated mainly in *in vitro* tissue culture conditions, hyaline cartilage has been shown to develop following treatment of human osteoarthritis with MSCs in the knee joint [Jo C. H. et al. 2014 Stem Cells 32: 1254-66]. Kota D.J. et al. reviewed the clinical studies performed employing MSCs [Kota D. J. et al. Int J Biochem Cell Biol. 2014 Aug 7;55C:1-10.]. They concluded that these cells are widely used. The authors were surprised to find that the diverse, increasing number of planned applications are not based on a well-defined therapeutic mechanism. The majority of the clinical studies involve autologous therapies.

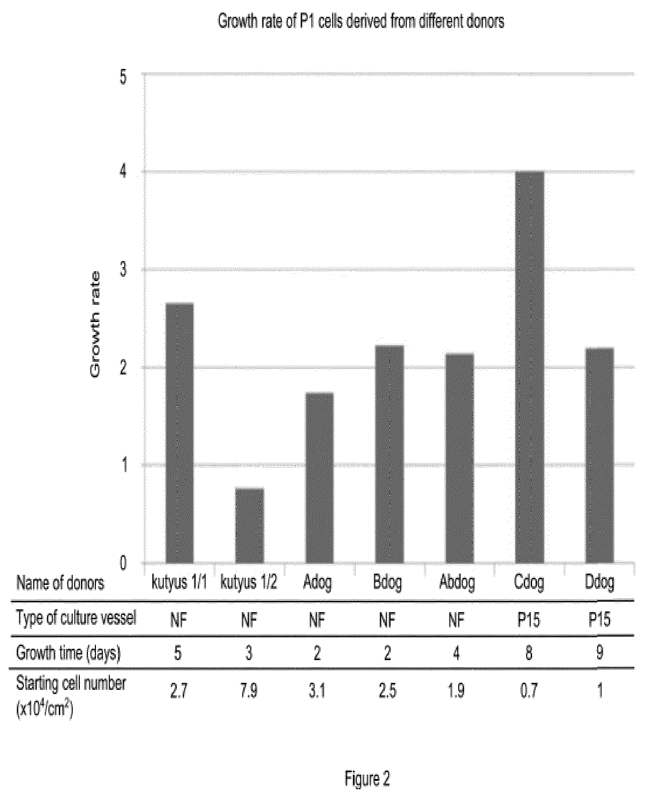

Furthermore, Chen Song et al. established [Chen Song et al. Genes & Diseases 2(1), (2015) 76-95.] that MSC hypertrophy poses a danger during cartilage regeneration, and adipose tissue-derived MSCs have a higher potential for hypertrophy than for chondrogenic differentiation (see Figure 2).

In US patent application No. US20130149285A**,** the inventors, **Lin, C. S. et al.,** describe the theoretical treatment of osteoarthritis in dogs (example 2) employing adipocyte-derived stem cells (ADSC). Their therapeutic regimen prescribes the administration of 5 million ADSCs in 0.5 ml PBS into every treated joint for a 20-kg dog. The results would be evaluated according to the Cincinnati Orthopedic Disability Index via the observation the movement of the dogs. The inventors describe an actual treatment for bone fracture in dogs. As a theoretical possibility only, besides many other diseases, they mention elbow dysplasia as well; however, they do not describe the method of culturing and formulation. As a general recommendation, collection of cells from adipose tissue (e.g. following liposuction) is suggested after washing, cutting, and appropriate treatment. Though the inventors mention the advantages of a biodegradable scaffold - among others that of a glycosaminoglycan - in general (paragraph [0083]), they do not describe its application.

To summarize the above, repairing the injured articular cartilage presents one of the greatest challenges to regenerative medicine due to the poor self-regenerative capacity of this tissue. Despite advances in orthopaedic surgery, autologous cell therapeutic methods - especially autologous chondrocyte transplantation - have gained an increasing role. However, these methods lead to dubious results as yet, and the repair of more severe cartilage injuries could only be assumed employing appropriate scaffolds.

Hoffman A. M. and Dow, S. W. ["Concise Review: Stem Cell Trials Using Companion Animal Disease Models" Stem Cells Translational and Clinical Research, 2016] review the studies based on stem cell therapeutic methods performed on domestic animals and pets, which can thus be used as models for human studies. The authors review the results of experiments performed on animals to treat osteoarthritis and conclude that in the case of treatment with adipose tissue-derived MSCs the improvement lasted for 3-6 months, though, they admit that the condition of the animals was in general not monitored beyond this time. In general, autologous treatment was employed. However, the authors state that though current treatments are promising regarding biological activity, further placebo-controlled studies are necessary.

Dysplasia is a typical cause of articular cartilage damage in dogs and other large animals such as horses. This disease is mainly, though not exclusively, a hereditary, genetic disease and not a result of injury. Thus, the obvious choice of using autologous MSCs does not necessarily represent a final solution since the MSCs carry the genetic defect; therefore, the cartilage developing from it will also be defective. Nevertheless, autologous transplantation, which is effective for 6-8 months and then has to be repeated, is an established procedure in the USA. We unexpectedly discovered a method with which the result of the treatment remains permanent, and the cured or significantly improved condition persists even after 12 months of observation. We unexpectedly recognized that administering a mixture of allogeneic donor-derived, cultured MSCs to the animals does not only decrease the risk of an ineffective treatment but stably increases the persistence of treatment effects, and even a single treatment may be sufficient to ensure long-lasting effects. Furthermore, the preparations, being mixtures of cells, can be standardized, stored, and the patients can be treated very soon after the treatment decision has been made.

The demand for a simple and effective treatment of elbow dysplasia or equivalent diseases with adipose-derived, allogeneic MSCs still persists.

The invention provides a solution to this problem.

### BRIEF DESCRIPTION OF THE INVENTION

The invention relates to a composition
for the treatment of an injury of the articular cartilage, preferably degeneration of the articular cartilage in a mammalian subject, wherein the composition comprises
- mesenchymal stem cells (MSC) obtainable by a method comprising isolating and culturing mesenchymal stem cells from at least two donor subjects of the mammalian species, the donors different from the subject to be treated, wherein the donor subjects are preferably diagnosed as free of dysplasia and optionally free of known diseases,
   and
- anionic nonsulfated glycosaminoglycan, preferably hyaluronic acid, preferably from 0.01% to 2% by weight, particularly from 0.1% to 1% by weight.

The composition is for use within up to 72 hours, 48 hours or preferably within 24 hours of the preparation of the compound. Preparation of the compound is to be understood as adjusting cell composition, adding all components and filling the composition into a syringe, the time period until use is calculated from the time point when this stage has been reached.

The joint injury is preferably an injury of the articular cartilage, preferably degeneration of the articular cartilage.

The joint injury is preferably an injury of the articular cartilage selected from joint dysplasia, articular cartilage detachment associated with osteoartritis, articular cartilage wear associated with osteoarthritis, cartilage wear associated with aging, traumatic cartilage detachment.

The injury of the articular cartilage is preferably an injury of the articular cartilage in the limbs. According to this embodiment the injury of the articular cartilage may be an injury of the articular cartilage of any of the following: hock, ankle, elbow, knee, shoulder, hip. The subject preferably has joint dysplasia (is dysplastic). The dysplastic subject preferably has clinical dysplasia.

The donor subjects are preferably healthy donor subjects, wherein no dysplasia, preferably no dysplasia without symptoms and/or no clinical dysplasia can be detected, preferably none of the foregoing can be detected.

The mesenchymal stem cells (MSC) are preferably obtained from the visceral adipose tissue of the donor subjects.

The method for the preparation of the MSCs preferably comprises at least isolation, cell culture and passage.

The amount of MSCs used for a single joint is preferably 10⁶ - 10⁹, preferably 1x10⁶ - 5x10⁷, in particular 5x10⁶ - 2x10⁷ MSCs.

The composition optionally comprises a biodegradable polymer matrix, preferably an anionic nonsulfated glycosaminoglycan, highly preferably 0.1-1% by weight of an anionic nonsulfated glycosaminoglycan, particularly hyaluronic acid.

Highly preferably the composition is for use within 24 hours.

The composition according to the invention is preferably for use in the treatment of an injury of the joint, preferably for the treatment of joint dysplasia, wherein the composition is obtainable by culturing a pool of MSCs derived from the visceral adipose tissue of 2, 3 or 4 healthy donor subjects and adding nonsulfated glycosaminoglycan to the culture.

The joint injury is preferably articular cartilage injury.

The joint injury is preferably joint dysplasia. The subject preferably has joint dysplasia (is dysplastic). The dysplastic subject preferably has clinical dysplasia.

Highly preferably the amounts of MSCs obtained from each donor subject are essentially the same or differ from the same up to ±30%, preferably ±20% or ±10%.

Preferably the joint dysplasia is elbow dysplasia, knee dysplasia, hip dysplasia or shoulder dysplasia or an injury of a cartilage, bone or ligament.

Highly preferably the joint dysplasia is a hereditary joint dysplasia in a horse or in a dog.

The composition according to the invention comprises or is obtained (prepared) from a pool or fraction of MSCs derived from a plurality of subjects.

In a preferred embodiment, the MSCs are from adipose tissue obtained from abdominal surgical waste, higly preferably MSCs are derived from the visceral adipose tissue obtained from ovariectomy of female dogs.

The donor subject may be identified by any genetic markers, e.g. nucleotid polymorphism (snp).

According to a preferred embodiment, the polymer matrix present in the composition is suitable to bind the MSCs and/or to maintain the viability of the MSCs and/or to stabilize the MSCs.

In a preferred embodiment, the polymer matrix is a glycosaminoglycan matrix, preferably hyaluronic acid matrix, highly preferably hyaluronic acid (HA) without crosslinking. Preferably the HA is present in the form of a salt, e.g. in the form of sodium-hyaluronate. Preferably the composition comprises the hyaluronic acid in a buffer, e.g. phosphate buffer and preferably in saline, e.g. isotonic saline.

In a preferred embodiment the polymer matrix, preferably the glycosaminoglycan matrix, highly preferably the hyaluronic acid matrix is present in the composition in a ratio of 0.01-5%, highly preferably 0.1-3% or 0.2-2%, most preferably 0.1-1% or 0.2-0.8%, e.g. 0.5%.

Highly preferably the mammalian species is the dog and
the composition comprises a total amount of 5 to 20 million MSCs isolated from two donor subjects different from the subject to be treated, in a donor subjects ratio of 0.4:0.6-0.6:0.4, in a suspension of 0.5-1.5 ml comprising from 0.2 to 0.8% hyaluronic acid and wherein the composition is formulated for use in administration into the joint.

Highly preferably the mammalian species is the horse,
the adipose tissue is adipose tissue of the regio inguinalis, which is preferably taken out under anesthesia, the donor is an allogeneic donor, and
the composition comprises a total amount of 10 to 30 million MSCs isolated from two donor subjects different from the subject to be treated, in a donor subjects ratio of 0.4:0.6-0.6:0.4, in a suspension of 1-2.5 ml comprising 0.2 to 0.8% hyaluronic acid, and wherein the composition is formulated for use in administration into the joint.

In a preferred embodiment, the composition is formulated for use in administration into the area affected by the elbow dysplasia in the mammalian patient. Preferably the composition is injectable.

According to a preferred embodiment the composition is formulated for use in administration into the joint affected by an injury, preferably by joint dysplasia in the mammalian patient. In another preferred embodiment the composition is formulated as an implant to be administered to come into contact with the joint, preferably the injured surface of the joint. Preferably the composition is injectable.

In a preferred embodiment the mammal is a dog.
The invention relates to a method for the preparation of a composition for the treatment of a joint injury, preferably joint dysplasia in a mammalian patient,
wherein
cultured mesenchymal stem cells (MSCs) are obtained by
(a) isolating portions of visceral adipose tissue from a plurality of subjects of the same species, wherein the subjects are different from the mammalian patient and then
(b) processing said portions by at least removing fat,
(c) obtaining said mesenchymal stem cells (MSCs) from said portions and culturing the cells;
(d) portions of a given or defined amount(s) or portions with a given or a defined number(s) of cells of cultured cells obtained from said cultured MSCs are stored, preferably frozen (preferably in such a way that every cell of each portion is cultured from cells obtained from the very same subject),
(f) the stored portions of MSCs are cultured prior to the preparation of the composition,
(g) a pool of cultured MSC portions is formed, wherein said pool comprises portions of MSCs obtained from the visceral adipose tissue of at least two healthy donor individuals of the mammalian species, wherein the donors are different from the individual to be treated,
(h) the pool of cultured MSCs is formulated into a composition by adding a biodegradable non-sulfated glycosaminglycan suitable to bind the MSCs and/or to maintain the viability of the MSCs and/or to stabilize the MSCs.
Alternatively, the steps may be carried out as follows:
(f') a pool or fraction of MSCs derived from a plurality of subjects after storage is prepared,
(g') the pool or fraction of MSCs is cultured,
(h') the cultured MSCs are formulated into a composition by adding a biodegradable nonsulfated glycosaminoglycan polymer matrix suitable to bind the MSCs and/or to maintain the viability of the MSCs and/or to stabilize the MSCs.

The joint injury in the above described methods is preferably an injury of the articular cartilage. The joint injury is preferably joint dysplasia. The patient preferably has joint dysplasia (is dysplastic). The patient with joint dysplasia preferably has clinical dysplasia.

The patient is preferably a non-human mammal.

Preferebly the invention relates to a method for the preparation of a composition for the treatment of joint dysplasia in a non-human mammalian patient, wherein prior to preparing the pool or fraction of MSCs obtained from a plurality of subjects
a) portions of visceral adipose tissue are isolated from a plurality of subjects different from the subject to be treated, wherein the plurality of subjects belongs to the same species,
b) processed portions of adipose tissue containing cells are provided by processing the portions of visceral adipose tissue by removing fat,
c) mesenchymal stem cells (MSC) are obtained from the processed portions of adipose tissue, preferably by using collagenase, allowing the cells to sediment and taking up again, in particular re-suspending the cells, preferably using a buffer comprising a balanced saline, e.g. Hanks buffer,
d) the MS cells are cultured, preferably to at least 70%, 80% or preferably 90% confluency, the cells are passaged at least once, are cultured again after passage, preferably to at least 70%, 80% or preferably 90% confluency,
e) portions of a given or defined amount(s) or portions with a given or a defined number(s) of cells of cultured cells obtained from the individual portions of adipose tissue are brought (separately from each other) into long term storage form (so that that every cell of each portion is cultured from cells obtained from a given subject), preferably they are frozen in fluid N₂, and stored.

According to a preferred embodiment the MSCs are obtained from the adipose tissue of viscera. Highly preferably MSCs are derived from abdominal surgical waste, e.g. from adipose tissue from the waste of neutering. Highly preferably MSCs are derived from inguinal adipose tissue.

In preferred embodiments the mammal is a herbivore, omnivore or a carnivore. According to a preferred embodiment the mammal is a domestic animal. According to a preferred embodiment the mammal is a canine, feline, equine, a ruminant or a porcine animal, e.g. a dog, horse, cat, cow, swine, sheep, camel, etc. Highly preferably the mammal is a horse. Highly preferably the mammal is a dog.

In a preferred embodiment the pool comprises MSCs from at least two or at least three mammalian subjects. Highly preferably the pool comprises MSCs from two mammalian subjects. Highly preferably the ratios and/or amounts of the cells from each mammalian subject present in the pool are essentially equal or almost equal, optionally differ from equal (50-50% or 1:1 ratio) up to 30%, 20% or 10% based on either the component present in the lower ratio or on the component present in the higher ratio.

In a further embodiment the pool comprises MSCs derived from at least 2, 3, 4 or 5, preferably 3-20, 4-15 or 5-10 mammalian subjects. Highly preferably the amount of the component present in the lowest ratio differs from the amount of component present in the highest ratio at a maximum of 80%, 70%, 60%, 50%, 40%, 30%, 20% or 10%.

Highly preferably the amounts of MSCs from each donor subject are essentially the same or differ from the same up to ±30%, preferably ±20% or ±10%.

Highly preferably the cell ratios in the pool given above are the cell ratios in the composition as well.

According to a preferred embodiment the pooled fraction is obtained by the following steps:

MSCs from a plurality of donors for the treatment of a joint affected by elbow dyplasia are thawed so that 0.1-15 million cells (1 x 10⁶ - 1.5 x 10⁷ cells) are used as starting amount for the composition for the treatment of a single joint.

The cells are diluted with cell culture medium (see protocol for MSC isolation) and are plated to a culture vessel in a density of 10⁴ cell/cm² (e.g. 1.5x10⁶ cells are plated onto a Petri dish).

P1 cells are cultured for e.g. 2-20 days, preferably 5-15 days, highly preferably 7-14 days, e.g. 9-13 days and the medium is changed every day to every 7 days, preferably every 2-5 days or every 2-3 days, preferably every 3 days. P2 cells are kept in culture for 2-4, highly preferably for 3 days after thawing and before transplantation.

Cells are detached from the vessel wall after culturing. Detachment is preferably carried out by using an enzyme, e.g. a peptidase or proteinase, e.g. trypsin. Cells are sedimented and then suspended in a buffer, preferably in PBS. Cells are then counted.

According to an alternative, cultured cells derived from multiple donors are mixed, preferably in almost equal ratios, preferably in a ratio of 1:1 or in a ratio that differs thereof up to 50%, preferably up to 40%, 30%, highly preferably up to 20% or 10% based on the component present in the highest amount.

According to a preferred alternative cultured cells from two donors are mixed, preferably in a ratio of 0.1:0.9-0.9:0.1 or 02:0.8-0.8:0.2 or 0.3:0.7-0.7:0.3, highly preferably in a ratio of 0.4:0.6-0.6:0.4 or in almost equal ratios, highly preferably in a ratio of about 1:1.

The mixture of cells is sedimented, preferably centrifuged.

Supernatant from the medium is disposed of after sedimentation and the cell sediment is suspended in a polymer matrix, preferably in a glycosaminoglycan matrix, preferably in hyaluronic acid. Highly preferably 0.05-5% or 0.1-1%, highly preferably 0.2-0.8% or 0.4-0.6%, in particular 0.5% of hyaluronic acid is used.

The hyaluronic acid is highly preferably hyaluronic acid without crosslinking. Preferably HA is present in the form of a salt, e.g. in the form of sodium-hyaluronate. The composition preferably comprises the hyaluronic acid in a buffer, e.g. phosphate buffer and preferably in saline, e.g. isotonic saline.

The hyaluronic acid is highly preferably a low molecular weight hyaluronic acid.

The volume of the hyaluronic acid solution is preferably 0.5-10 ml or 0.5-5 ml, preferably 1-3 ml, highly preferably 0.5-2 ml or 1.2-2.5 ml or approximately 1-2 ml.

For the treatment of a single joint 1-500 million cells, preferably 1-100 million cells or 5-100 million cells, highly preferably 2-50 million cells or 5-50 million cells or 10-50 million cells or 10-30 million cells, in particular approximately 10-20 million cells (preferably 1x10⁶ - 1 x 10⁸, 5x10⁶ - 1 x 10⁸, 2x10⁶ - 5 x 10⁷, 5x10⁶ - 5 x 10⁷, 1x10⁷ - 5 x 10⁷, 1x10⁷ - 3 x 10⁷ in particular approximately 1-2x10⁷ MSCs) are used.

Highly preferably this amount is used for the treatment of a single joint in dogs, for example for the treatment of a single joint of a single limb or shoulder or hip or elbow and hock (ankle) or knee.

The weight of the dog is for example 5-60 kg or 10-50 kg, preferably 15-50 kg or 20-45 kg or 10-40 kg, highly preferably the animal, preferably dog is of a small size, medium size or large, e.g. its weight is 10-20 kg, 10-30 kg or less; 20-40 kg; or 30-50 kg or more.

In a highly preferred embodiment, in case of a dog, portions of MSCs isolated from two or three, e.g. three dogs which are different from the dog to be treated ,are used in equal ratios or in ratios that differ thereof up to 30% based on the component present in the highest amount, using a total amount of 5-100 million cells per joint in a suspension comprising 0.1-1% hyaluronic acid, in a volume of 1-5 ml.

In a highly preferred embodiment, in case of the dog, MSC portions isolated from two dogs which are different from the dog to be treated are used in a ratio of 0.4:0.6-0.6:0.4, using a total amount of 2-50 million, e.g. 2-30 million or 5-50 million cells per joint in a suspension comprising 0.2-8% hyaluronic acid, in a volume of 1-5 ml, preferably 0.4-2.5 ml or 0.5-2 ml.

In case of a larger animal, administration of a larger amount of cells may be enough or needed, whereas in case of a smaller animal a smaller amount of cells may be enough or needed.

For example, for smaller animals, e.g. dogs 2x10⁶ - 1x10⁷ cells, e.g. 2x10⁶ - 5x10⁶ cells are used, for medium sized animals, e.g. dogs 5x10⁶ - 2x10⁷ cells, e.g. 5x10⁶ - 1x10⁷ cells are used and for large dogs and other large animals 1x10⁷ - 5x10⁷, e.g. 1,5x10⁷ - 2,5x10⁷ cells are used.

Preferably, 0.5-2 ml suspension is used.

Highly preferably, for smaller animals, e.g. dogs 0.5-1 ml suspension, for medium sized animals, e.g. dogs, 0.8-1.5 ml suspension and for large dogs and other large animals, 1-2 ml suspension is used.

According to a further embodiment it is not necessary to increase the amount of the cells to the extent of the increase of the weight of the animal, e.g. a quadratic change in the weight of the animal may be assigned to a linear change in the amount of the cells.

The invention also relates to a treatment method, wherein the composition according to the invention or the composition obtained according to the invention is administered to an animal suffering from joint dysplasia.

According to a preferred embodiment the composition is administered into the joint affected by dysplasia of the elbow joint in the mammalian patient or is administered to contact the joint.

According to a preferred embodiment the composition is formulated for use in administration into a cartilage, preferably formulated in a manner identical to a known composition for use in administration into a cartilage.

Highly preferably the composition comprising the MSCs and the matrix comes into contact with the injured surface of the joint.

In a preferred embodiment the mammal is a dog.

### DEFINITIONS

As used herein, *"Stem cells"* refer to undifferentiated or not fully differentiated cells that are, on the one hand, capable of performing - in some cases a limited number of - divisions for self-maintenance and, at the same time, can generate one or more differentiated cell types.

"*Tissue stem cells"* are multipotent stem cells that have at least partially remained undifferentiated during ontogenesis - preferably until adulthood - and are capable of differentiating into preferably a limited number or type of tissues, preferably into tissues or cells that are in contact with them.
The role of tissue stem cells is to ensure the renewal of the tissues of the organism and the replacement of dead or aging cells.

*"Mesenchymal stem cells"* (MSCs), as used in the description, are multipotent - preferably stromal, that is of supportive tissue origin and/or localization - tissue stem cells that are
- adherent (capable of attaching to the surface of the vessel);
- capable of differentiating into at least bone, cartilage, and adipose cells *in vitro* and are preferably
- CD105-, CD73-, and CD90-positive but do not carry surface markers characteristic of hematopoietic stem cells and progenitors and preferably do not carry surface markers characteristic of blood cell development lineages; that is, the MSCs are preferably CD45-, CD34-, CD14-, CD11b-, CD79a-, and CD19-negative.

As used herein, *"Stroma"* or "*supportive tissue"* refer to a tissue that associates with or surrounds a given structure of the organism, e.g. an organ or preferably its functional tissue *("parenchyma")* and has a role in mechanically supporting the structure or organ or preferably the parenchyma. Preferably, the stroma contains or mostly contains or consists of conjunctive tissue.

The *"Conjunctive tissue"* is a fibrous tissue that contains at least extracellular fibres, amorphous intercellular material (basic substance), and fixed and mobile cells where the fibres are embedded in the basic substance. Conjunctive tissues are, among others, the bone tissue, the tendon tissue, the cartilage tissue, and the adipose tissue. The conjunctive tissue supports and connects the internal organs, participates in bone formation, builds the walls of veins, connects muscles to bones, and in case of injury replaces other types of tissues.

As used herein, *"Adipose tissue"* refers to an animal conjunctive tissue that consists mainly of adipose cells (adipocytes) of mesenchymal origin, whose most important functions are energy storage, the protection of the organism from physical effects (cold, mechanical impact), and the cushioning of certain organs. Two types of adipose tissue can be distinguished: the yellow (also known as white) adipose tissue (which plays an important role in energy storage) and the brown adipose tissue (whose primary function is to generate body heat).

As used herein, *"Adipose cells"* or *"Adipocytes"* refer to cells that are specialized for the storage of energy in the form of fat in lipid vacuoles. Besides the presence of lipid vacuoles, adipocytes are preferably characterized by, among others, the expression of the following markers: PPARg, aP2 and HSL, Adipoq, Cebpa. Adipocytes can be white adipocytes characterized by, for example, their flat and peripheral nucleus, large vacuoles, and the secretion of adiponectin, rezistin, and leptin or can be brown adipocytes that are, for example, polygonal.

As used herein, *"Visceral"* organs refer to the internal organs of the organism, particularly to those that are located in the abdominal cavity and the chest (thoracic cavity), e.g. the respiratory system, the digestive system, the urinary system, the internal genital organs, the liver, the heart, or the stomach.

*"Visceral adipose tissue"* is the adipose tissue associated with or surrounding the "visceral" organs.

As used herein, *"Joint"* refers to a formation located at the intersection of bone ends of mammals that enables a type of movement defined by the geometry of the joint or of the joining ends of the bones and in which the bone ends are covered by a smooth surface, the "*articular cartilage",* and synovial fluid (synovia) is found in the joint cavity (cavum articulare), and the above are surrounded by a closed articular capsule (capsula articuris) the inner layer of which (membrana synovialis) secretes the synovial fluid while the outer layer (membrana fibrosa) is a fibrous conjunctive tissue which has a protective and mechanical role. The joints preferably contain articular connective tissue ligaments (ligamentum articulare) as well to keep the joints or the joining bone ends mechanically steady, which, in some cases (e.g. the shoulder joint), is also helped by the surrounding muscles. Joints generally have an articular fossa (fossa articularis) and an articular head (caput articulare) which fits into it. In more complex joints (e.g. the knee) the lack of geometric fitting between the two joining ends is solved by a mobile, crescent-shaped fibrocartilaginous structure (meniscus).

*"Hyaluronic acid"* (hyaluronate) is an anionic nonsulfated glycosaminoglycan, the copolymer of disaccharides composed of D-glucuronic acid and D-N-acetylglucosamine, in which the monomers are linked via alternating β-1,4 and β-1,3 glycosidic bonds (CAS No. 9004-61-9). It is typically water-soluble in its sodium salt (CAS No. 9067-32-7) or potassium salt (CAS No. 9067-32-7) form. The molecular weight of the polymers is typically in the range of 5 kDa to 20,000 kDa.

As used herein, *"High molecular weight hyaluronic acid"* (HA) typically refers to hyaluron acid with a molecular weight of about one thousand kDa or above while *"low molecular weight hyaluronic acid"* typically refers to hyaluronic acid below the molecular weight of one thousand kDa, preferably hyaluronic acid with a molecular weight of 0,8-8 x 10² kDa. Typically, high molecular weight HA refers to HA with a weaker inflammatory potential.

As used herein, *"Joint dysplasia"* refers to a hereditary disease of mammals (preferably large-sized mammals) in which the fitting of the joining (articulating) ends of the bones - preferably the articular head and the articular fossa - is not proper; it is defective or imprecise leading to the deformation, in certain cases to the partial (or total) dislocation of the joint. After a time, arthrosis develops between the cartilage of the articular head moving in and out and the edge of the articular fossa due to the *"clinically dysplastic"* state in the constantly deformed joint.

As used herein, an *"Individual"* or *"subject"* refers to a given animal being. Preferably, as used in the description, animal does not include human beings.

A *"Patient"* is an individual who/that is under medical diagnosis, observation, or treatment. The treatment can be preventive and/or curative. Preventive treatment refers to preventing or delaying the deterioration of the patient's condition. Curative treatment refers to improving the patient's condition.

In the appendices of the application, unless indicated otherwise, the singular form of a word - regarding its meaning - encompasses the plural form. Analogously, the article "a" in front of a noun, unless indicated otherwise, encompasses the plural of the noun.

In the description, the term *"comprises"* should be understood in a way that the thing that *"comprises"* something may contain other elements besides the elements listed, but it contains - by all means - the listed elements. The expression "comprises" encompasses the expression "*essentially consists of'* because this latter refers to the presence of other elements besides the ones listed, but these other elements do not contribute to the technical effect of the invention. In case of the expression *"consists of',* the thing that consists of something cannot contain other elements besides the ones listed, unless these are trivial elements to be implicitly included in the content of the thing that consists of something. The expression "comprises" can be limited to either of the expressions *"in essence consists of'* and *"consists of'.*

### BRIEF DESCRIPTION OF THE FIGURES

**Figure 1****.** shows the growth rate of P0 cells (cells after isolation, before passage) derived from different donors. Types of culture vessels: NF: 75 cm² flask, KF: 25 cm² flask, P15: 145 cm² petri dish. Growth time: the days indicated represent the time period (days) between plating and passage 1. Donor names: kutyus1/1, kutyus1/2, Adog, Bdog, Abdog, Cdog, Edog
**Figure 2****.** depicts the growth rate of P1 cells (cells after passage 1) derived from different donors. Types of culture vessels: NF: 75 cm² flask, P15: 145 cm² petri dish. Growth time: the time period (days) between passage 1 and passage 2. Donor names: kutyus1/1, kutyus1/2, Adog, Bdog, Abdog, Cdog, Edog
**Figure 3****.** presents the results of culturing MSCs from a given dog ("E-dog"), that is, the total cell number achieved by day 9 with protocols employing different medium changes. Interpretation of medium changes: - /+ 25 ml indicates that the medium was totally removed from the cells and 25 ml fresh medium was added; -/+ 5 ml and -/+10ml indicate that, of the 25 ml medium, only 5 ml and 10 ml were removed, respectively, then the medium remaining on the cells was supplemented with 5 ml and 10 ml fresh medium, respectively.
**Figure 4****.** demonstrates the effect of horse serum on the growth of MSCs until passage 1 (during period P0 and before period P1); in particular, it shows the growth rate of cells in the presence of horse serum at different ratios.
**Figure 5****.** displays the effect of horse serum on the growth of MSCs until passage 1 (during period P1); in particular, it shows the growth rate of cells in the presence of horse serum at different ratios.
**Figure 6****.** demonstrates the effect of horse serum on the growth of MSCs until passage 2 (during period P2); in particular, it shows the growth rate of cells in the presence of horse serum at different ratios.
**Figure 7****.** depicts the decrease in the number of cells following thawing of the cells due to centrifugation; cell number without centrifugation is considered 100%.
**Figure 8****.** displays the amount of dead and living cells following culturing in 0.5% hyaluronic acid in syringe for 24 hours at 4°C and at room temperature (RT) and a further 2 days in tissue culture.

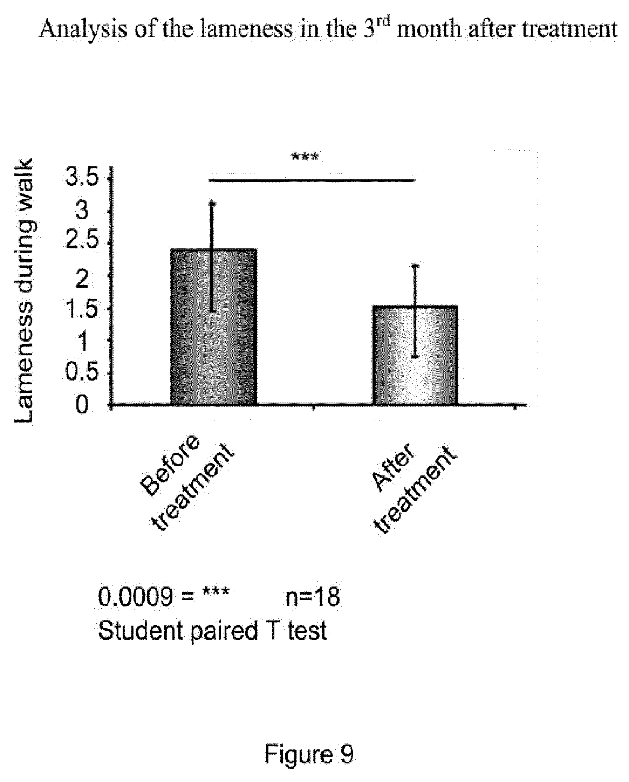

**Figure 9****.** demonstrates Student's paired t-test statistical analysis of the lameness of 18 dogs, characterized by scores, before treatment and three months after the treatment.

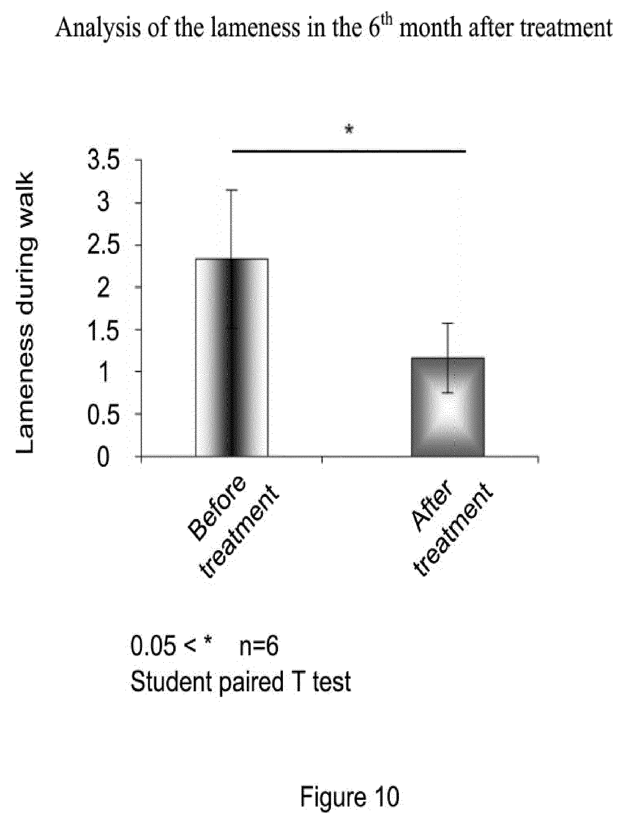

**Figure 10****.** shows Student's paired t-test statistical analysis of the lameness of 6 dogs, characterized by scores, before treatment and six months after the treatment.
**Figure 11****.** displays data of 40 dogs regarding lameness at walk, with statistical evaluation (± SEM. *** significance <0.001, n=40 follow-up after transplantation: 6, 9, 12 months.)

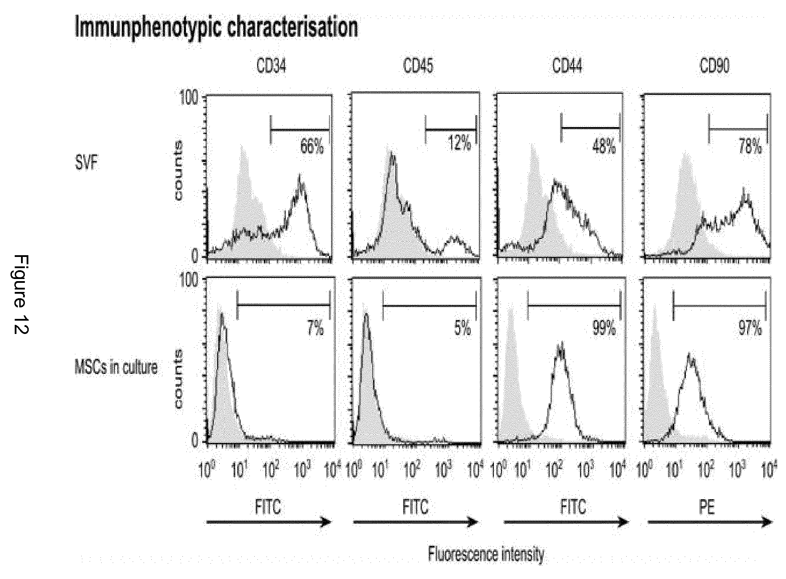

**Figure 12****.** demonstrates the analysis of cell surface markers of MSCs (passage 2). The markers were analysed using FITC-conjugated rat anti-mouse/human CD44 (BioLegend, 1.2 ng/20 µl) and PE-conjugated rat anti-canine CD90 (eBioscience, 6 ng/ 20 µl) antibodies.

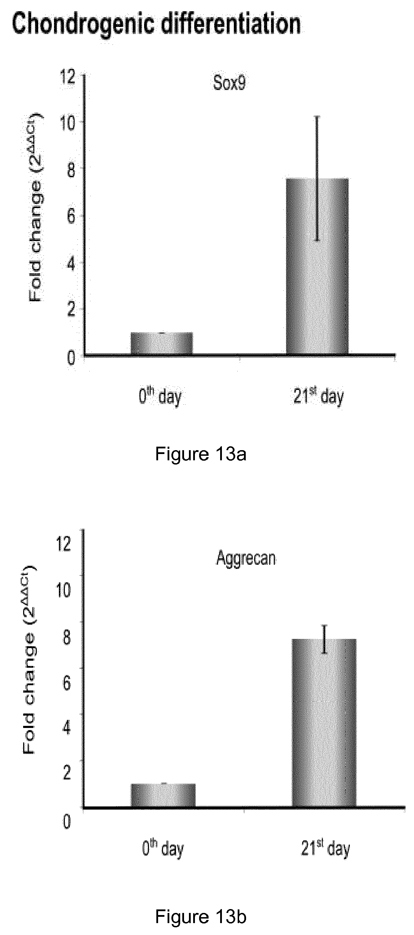

**Figure 13****.** shows chondrogenic differentiation of passage 2 MSCs. Chondrogenic differentiation was induced in three-dimensional pellet culture. The expression of the *Gapdh, Sox9,* and *Aggrecan* genes was determined using quantitative real-time PCR. All results were normalized to the expression of the housekeeping *Gapdh* gene. Figure 13a depicts the expression of the *Sox9* gene and Figure 13b depicts the expression of the *Aggrecan* gene on days 0 and 21.

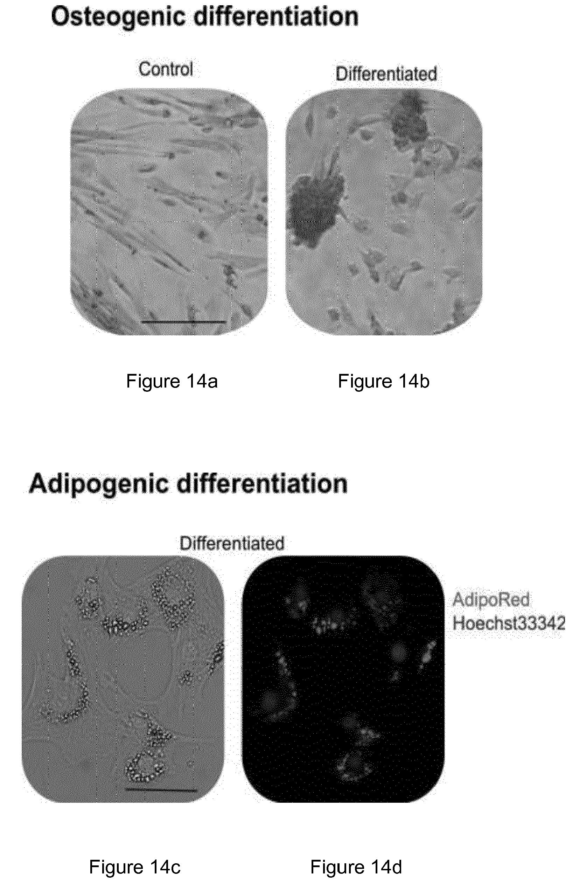

**Figures** 14a and 14b of **Figure 14****.** display the osteogenic differentiation ability of MSCs, while Figures 14c and 14d demonstrate the adipogenic differentiation ability of MSCs.

In case of osteocyte differentiation, passage 3 cells were differentiated in differentiation medium, and on day 21 the cells were washed with PBS and fixed with 8% formaldehyde for 15 minutes. The experiment reveals the appearance of potassium deposits stained red in the treated cell culture, indicating osteogenic differentiation.

In case of adipogenic differentiation, following 4 days of treatment, the cells were washed with PBS and fixed with 8% formaldehyde for 15 minutes. Red staining reveals the accumulated fat droplets in the samples.

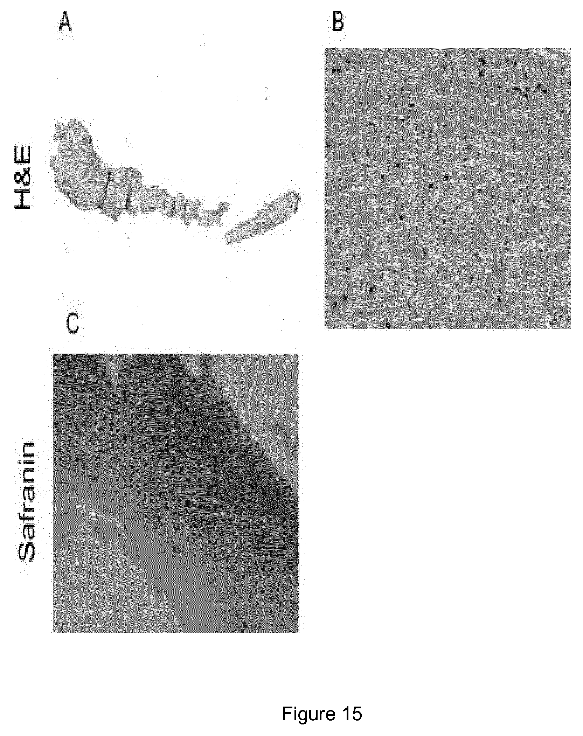

**Figure 15****.** demonstrates the histochemical analysis of cartilage biopsy taken from the regenerated area, following paraffin embedding and sectioning. Figure 15a: The entire biopsy at 25x magnification. Figure 15b: A glycoseaminoglycan-containing matrix is visible around the chondrocytes. Figure 15c: Analysing the presence of proteoglycans.

### DETAILED DESCRIPTION OF THE INVENTION

The articular cartilage may be damaged by several diseases, traumas, or environmental factors. The disease may be of genetic origin or may arise due to environmental factors such as obesity, aging, heavy duty and result in osteoarthritis (joint arthrosis), joint dysplasia, cartilage wear, mechanical injury of the cartilage, or cartilage detachment. These diseases cannot always be clearly distinguished, e.g. arthrosis of the joint may manifest as joint dysplasia. Furthermore, wear and mechanical injury of the cartilage may also accompany the primary disease.

The function of the articular cartilage is to dampen vibration during the movement of the animal (or human) and, at the same time, prevent the bones from rubbing against each other during the movement of the joint.

Loss of the articular cartilage leads to limited movement of the extremities, partly because of the developing pain and partly because of the loss of cartilage function.

One of the most prevalent diseases involving articular cartilage injury is osteoarthritis. With time, patients afflicted by this disease lose the functionality of their joints. Osteoarthritis leads to damage of the cartilage tissue between the bones in the initial phase of the disease. In case of osteoarthritis, the cartilage thins out, and this process leads to abnormal thickening and widening of the underlying bone, which finally manifests in the form of sharp spikes.

In advanced osteoarthritis, the cartilage can become practically detached, and in such cases the bones can rub against each other.

In a preferable embodiment, the injury emanates as a result of joint dysplasia.

Dysplasia refers to the abnormal anatomy of the articulating bones. As a result, the bone ends mechanically damage the cartilage, and thus cartilage wear, cartilage detachment or degenerative cartilage diseases (e.g. osteoarthritis or arthrosis) develop. This can also develop due to a genetic effect when the chondrocytes are not able to produce a proper matrix, thus, they are not anchored. It can also develop as a result of joint infection, inflammation. In the latter case, the cause and effect cannot be discerned. The former may develop as a result of surgical intervention or local treatment of the joint (e.g. administration of an injection).

Joint dysplasia is a genetic disease caused by a combination of paternal and maternal genes, which affects the joints in mammals. In certain cases, joint dysplasia involves imprecise or uneven fitting of the joint, which results in an uneven distribution of the weight of the mammal on the articular cartilage. In areas under increased load, the cartilage covering the bones may be damaged resulting in the deterioration and wear of the cartilage and the underlying bones.

As the disease progresses, cartilage wears down in the deformed, in more severe cases continuously dislocated joint. Furthermore, articular inflammation and, after a time, cartilage degeneration, osteophytes, degenerative joint deformation, in sum, joint arthrosis develops. Partial dislocation of the joint and the resulting inflammation can themselves cause movement defects, but real pain and lameness is induced by cartilage wear and the development of arthrosis.

In general, the signs of joint dysplasia appear in the developing individuals, during growth, or in early adulthood. In the case of dogs, this is typically the age of 5-8 or 5-12 months, possibly before the age of 2 or 3 years. Symptoms of the disease may appear at any later age.

In the case of horses, cartilage injury is treated frequently.

### Assessment of dysplasia

Screening tests with which ambiguous parents are excluded from breeding play a pivotal role in the prevention of hip dysplasia. It is reasonable to pre-screen animals after birth so that dogs with unfavourable extremity development can be helped in time. Extremity preferably refers to the hip or the elbow. The suggested time of pre-screening is the age of six months, while final screening should be performed preferably between the age of one and two years.

Joint dysplasia can be detected using X-ray, e.g. CT.

During the assessment of dysplasia, basically two conditions can be distinguished:
- 1. Improper joint structure can be verified by X-ray in the animal carrying the disease without other symptoms ("asymptomatic dysplastic" animal).
- 2. Improper joint structure can be verified by X-ray in the animal carrying the disease with movement defects due to the disease ("clinically dysplastic" animal).

The treatment provided in the invention can be employed mainly in the latter condition.

An animal dysplastic from a breeding point of view (1.) can obviously become a clinically dysplastic animal later (2.); however, an asymptomatic dysplastic animal may in a fortunate (mild) case live all its life without lameness or problems.

Surgery is indicated particularly in the case of clinically dysplastic animals. At the same time, MSC therapy can replace surgery or - in more severe, but operable cases - it can complement surgery.

Joint dysplasia is particularly common among dogs.

Based on the joint affected, several dysplasias can be distinguished.

### Hip dysplasia

Hip dysplasia is a hereditary disease inherited from the parents, grandparents. It can manifest in male and female dogs as well. Its appearance is more frequent in large dog breeds. The symptoms of hip dysplasia are: the dog is not willing to stand up, it walks with difficulty, with tight, short steps. The disease can be first diagnosed with X-ray within the frame of hip dysplasia screening at about the age of 10 months.

### Knee dysplasia

Knee dysplasia, a congenital dislocation of the patella, affects small dog breeds. If the dislocation resolves spontaneously, the dog is able to walk again. The disease is hereditary. The dislocation of the patella involves deformation, and this may lead to increased wear of the joint, arthrosis, ligament rupture, and lameness.

### Elbow dysplasia

Elbow dysplasia is a hereditary developmental disease found mainly in large dog breeds, which manifests at around the age of 4 and 8 months and, if left untreated, leads to irreversible damage of the joint, arthrosis.

Screening for elbow dysplasia is of great importance. Deformation and pain often appear late, when treatment is less effective, and the deformations of the elbow joint are irreversible.

Examples of large dog breeds include German Shepherd, Golden Retriever, Rotweiler, Caucasian Shepherd Dog, Irish Wolfhound, Labrador Retriever, Saint Bernard, Belgian Shepherd, and the Dog.

### Shoulder dysplasia

Shoulder dysplasia in dogs is the dysplasia of the scapular glenoid and the humeral head that constitute the shoulder joint. Due to the defective fitting, shoulder arthrosis, a degenerative joint disease with cartilage wear and osteophytes, may develop.

According to the present invention, elbow dysplasia is treated with a mixture of allogeneic stem cells derived from the adipose tissue of several (2-10) healthy donors. We found that the allogeneic MSCs (derived from a different donor) are not rejected by the treated animals. We presume that this is due to the strong anti-inflammatory and immune response-inhibiting effects of the MSCs.

According to the present invention, allogeneic MSCs are taken from inguinal (horse) or visceral (dog) adipose tissue employing a humane procedure or from surgical waste. For example, we collect them from visceral adipose tissue that is generated as waste during the ovariectomy of healthy female dogs (removed to clear the surgical site).

We discovered that this procedure has several advantages: 1) the sick dog does not have to be exposed to a relatively invasive procedure (adipose tissue removal) before transplantation; 2) the allogeneic adipose tissue reduces the chance of the stem cells carrying the same genetic disease; 3) the mixture of the donor stem cells further reduces the chance of potentially transferring non-screened genetic disorders into the patient and; 4) the mixed stem cell pool enables the generation of a preparation containing a large amount of MSCs which ensures the application of the standard transplant without further characterization.

Since MSCs only survive for a short period (a few hours) following separation from the solid support (in *in vitro* cultures the detachment from the wall of the vessel, in *in vivo* environment the separation from the conjunctive tissue), transfer of the MSCs to the site of transplantation poses difficulties. For this reason, the MSCs are suspended in a physiological solution containing 0.5% hyaluronic acid (HA) and are transported in a syringe, in "ready for use" condition to the veterinarian who performs the treatment. Hyaluronic acid is an anionic nonsulfated large molecular weight glycosaminoglycan (it can reach one million Daltons), one of the basic components of the conjunctive tissue and the extracellular matrix. Since it can function as a scaffold, as a natural support for stem cells, the cells can attach to it. By employing it, the life span of MSCs in the suspension increases advantageously.

As an additional advantage, in case of osteoarthritis, the injection of hyaluronic acid into the joint provides a natural "lubricant", which decreases inflammation resulting from loss of cartilage. Last but not least, HA enhances the chondrogenic differentiation of multipotent MSCs.

MSCs display CD44, a hyaluronic acid receptor, which enhances the binding of hyaluronic acid.

Hyaluronic acid is employed highly preferably in the form of sodium hyaluronate, in isotonic saline and at physiological pH. Hyaluronic acid is employed highly preferably in the form of a 0.5% solution. Thus, hyaluronic acid is employed highly preferably in the form of a preparation that is similar to synovial fluid.

It is not only ED of dogs that can be treated with the method above but also cartilage, tendon, and bone injuries of cats and horses (Nixon AJ et al. 2008. Am. J. Vet. Res. 69:928-37); therefore, we treated horses as well, and we also wish to include other animals in the development of the therapeutic protocol.

Below, the solution is presented more concretely, partly through examples, maintaining that alternative embodiments are possible.

According to a preferred embodiment, MSCs are isolated from adipose tissues of internal organs (visceral adipose tissue). Highly preferably, they are isolated from abdominal surgical waste, e.g. waste adipose tissue from ovariectomy. Highly preferably, they are isolated from inguinal adipose tissue.

Adipose tissue is processed preferably within 24 hours; cell viability and yield are not affected within this period of time.

Cells are preferably released with a collagenase solution. An example of this is provided in the description below, but professionals are aware that alternative methods exist. According to a preferred embodiment, a 0.1-1% collagenase solution is prepared in a "balanced salt solution", that is, in a solution imitating physiological conditions (e.g. in Hank's buffer; the amount depends on the amount of adipose tissue to be treated). Balanced salt solutions generally contain sodium, potassium, calcium, and magnesium ions, and chloride as anion or, possibly, other physiologically tolerated ions. Furthermore, they may contain nutrients for the cells, e.g. glucose, and buffer to set the physiological pH, which can be easily controlled by the professional.

After isolation, cells are cultured in a medium suitable for the culturing of mammalian cells, e.g. Eagle's minimal essential medium (EMEM) or its Dulbecco-modified version, DMEM [Dulbecco's minimal essential medium (EMEM)], or preferably in F12-supplemented DMEM (DMEM/F12). The media suitable according to the invention may contain, e.g. amino acids, salts (e.g. chlorides, sulphates, and phosphates of calcium, potassium, magnesium, and sodium), sugars, e.g. glucose, and vitamins (e.g. folic acid, nicotinamide, riboflavin, B12), etc. Further information about suitable media can be found in the review of Sato et al. [Sato, J. D. and Kan, M. 2001. Media for Culture of Mammalian Cells. Current Protocols in Cell Biology. 00:1.2:1.2.1-1.2.15. (Published Online: 1 MAY 2001; Published Print: OCT 1998)]. Detailed guidance about the methods of culturing mammalian cells can be found in the works of Phelan [Phelan, M. C. 2007. Basic Techniques in Mammalian Cell Tissue Culture. Current Protocols in Cell Biology. 36:1.1:1.1.1-1.1.18. (Published Online: 1 SEP 2007, Published Print: SEP 2007)] and Vinci V.A. et al. [Vinci, Victor A., Parekh, Sarad R. Eds. Handbook of Industrial Cell Culture: Mammalian, Microbial, and Plant Cells; Humana Press, 2003 ISBN 1588290328, 9781588290328].

According to an embodiment of the invention, the culture is preferably passaged at least once, perhaps twice or thrice.

The ready culture is brought into a condition that can be stored.

The skilled person is familiar with the methods of culturing, passaging, and storing cells [see e.g. **Phelan,** M. C. et al., 2007 and **Vinci,** V.A.et al., Eds. 2003, infra].

After storage, the individual fractions or doses are cultured, and several fractions from different animals are combined to produce the preparation.

According to a preferred embodiment, the combined fraction contains MSCs from at least two or at least three mammalian individuals.

According to further embodiments, the combined fraction contains MSCs from at least two, three, four, or five, preferably 3-20, 4-15, or 5-10 mammalian individuals. Highly preferably, the component at the lowest ratio differs from the component at the highest ratio at a maximum of 80%, 70%, 60%, 50%, 40%, 30%, 20% or 10%.

The number of different individuals and the ratio of cells derived from them can also be determined from the preparation by analysing the genetic material of the cells. The analysis can be performed using a genetic marker that is characteristic of a given individual, by identifying the difference from other individuals. A possible method of analysis is the "single nucleotide polymorphism", snp. Such methods are well known in the present state of technology [see e.g. Single Nucleotide Polymorphisms: Methods and Protocols; Editor(s): Anton A. Komar Series: Methods in Molecular Biology; Volume No.: 578 (2008); DOI: 10.1007/978-1-60327-411-1].

Highly preferably, the amount of stem cells derived from the different individuals in the combined fraction is basically the same or deviates from the same at a maximum of ±30%, preferably at ±20% or ±10%.

Highly preferably, the cell ratios given for the combined fraction above apply to the cell ratios of the preparation as well.

As preferable packaging, the preparation suspended in hyaluronic acid is filled into syringe and closed with a stopper. The syringes are packed, e.g. into sealable bags and paper boxes. The preparation thus packed is transported in cooled conditions in a polysterene box with a cooler block.

### Preparation for treatment

The aim of the experiments is to develop a therapeutic procedure to cure hereditary and traumatic degenerative cartilage diseases in dogs. During the process, dogs suffering from elbow, knee, shoulder, ankle or hip dysplasia are treated with mesenchymal stem cells, employing a minimally invasive procedure performed in narcosis. During the process, the stem cells are injected directly into the affected joint.

The animals involved in the experiment cannot be substituted, because we are developing a therapeutic procedure for exactly these animals, with which the incurable ED becomes curable, and recovery from other orthopaedic diseases can be accelerated with the help of a minimally invasive cell therapeutic procedure. The owners were informed that the animals will exhibit either improvement or unchanged condition at the end of the treatment, but euthanasia as a result of the procedure or the evaluation will in no way be necessary. The animals are involved in the experiments based on the consent and pronounced intent of the owners. Based on data from the literature, we expected that MSC transplantation will not have severe side effects either systemic or local. (Jo CH et al. 2014 Stem Cells 32: 1254-66). Isolation of stem cells from healthy donors belongs to the essence of the procedure because we wished to rule out the possibility that the tissue which develops from the own stem cells of an individual becomes as damaged as the original. Employing allogeneic donors is also supported by the fact that waste adipose tissue derived from abdominal surgery, e.g. from ovariectomy of female animals can be the source of stem cells without the need to perform a separate invasive surgery on either the donor or the patient animal.

Evaluation of the approved veterinary symptoms is based on the observations of the veterinarian and the owner: lameness in walking and trotting, pain to touch, pressure, range of motion, functional abilities, inflammation/need for pain relief medication, individual scoring of asymptomatic period from 1 to 5 (see example 6).

### EXAMPLES

### Example 1

### Experiments to define the conditions for the isolation and culturing of MSCs

Experiments were performed to define the appropriate conditions for the isolation and culturing of canine MSCs.

### Transport conditions for adipose tissue and isolation of MSCs

We found that adipose tissue removed from the abdomen of dogs can be, for example, placed at 4°C for 24 hours in DMEM without FCS (Fetal Calf Serum) and antibiotics or in Hank's solution and, subsequently, the same amount of cells can be isolated as from the freshly processed adipose tissue.

For example, with immediate processing, we isolated 2.69 x 10⁷ cells (100%) from 30.5 g adipose tissue, while we isolated 3 x 10⁶ cells (125%) from 2.8 g adipose tissue following its storage at 4°C for 24 hours.

### The initial growth of MSCs, before passage 1, depends on culture conditions

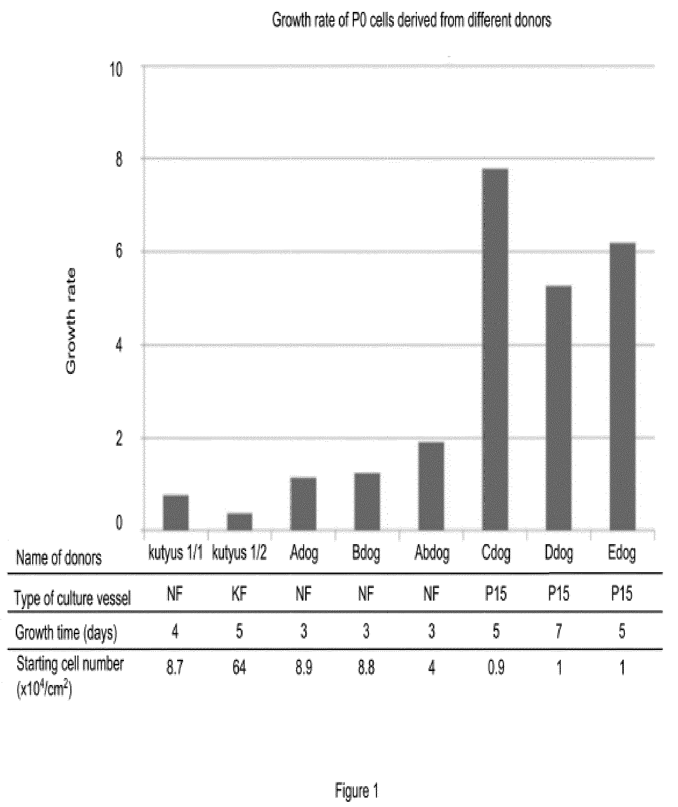

Experiments were performed to determine the growth rate of P0 cells (cells before passage) derived from different donors. The result of the experiment is shown in *Figure 1**.*

The figure demonstrates that several types of culture vessels were used (NF: 75cm² flask, KF: 25cm² flask, P15: 145cm² petri dish). Growth time indicates the time period in days between plating and the first passage. Donor names identify the dogs from which the cells were obtained.

The isolated cells are transferred into tissue culture. In this phase, the culture is designated P0. Growth rate (x) indicates the number by which the number of cells plated initially is multiplied at the end of P0, that is, before passage.

Based on the experiment above, we concluded that growth rate depends on the donor, the initial number of cells, and the growth time.

The preferable initial cell density is 1 x 10³ - 2 x 10⁴ cells/ cm², a more preferable is 5 x 10³ - 1.5 x 10⁴ cells/ cm² and the highly preferable is 0.8-1 x 10⁴ cells/cm² or about 7 x 10³ cells/cm².

The time of the first passage, particularly at the given initial cell number, is preferably 3-9 days or 4-8 days, preferably 5-7 days.

Highly preferably, 12-1.7 x 10⁶ cells or 1.35-1.5 x 10⁶ cells are used in 15 cm petri dishes.

The initial cell number is also influenced by whether the cells thus diluted retain their chondrogenic differentiation ability. Differentiation ability decreases with the increasing number of passages. This requires further testing.

Cells are cultured preferably until 70% confluency, highly preferably until 80% confluency, and even more preferably until at least or about 90% confluency.

For example, P0 cells reaching 90% confluency were detached from the bottom of the culture vessel using trypsin solution, counted and, to achieve further growth, diluted and transferred to another culture vessel. This cell culture is designated P1.

### Growth of cells derived from different donors after the first passage

Experiments were performed to determine the growth rate of P1 cells (cells after the first passage) derived from different donors. The result of the experiment is shown in *Figure 2**.*

The figure demonstrates that several types of culture vessels were used (NF: 75cm² flask, P15: 145 cm² petri dish). Growth time indicates the time period in days between the first passage and the second passage. Donor names identify the dogs from which the cells were obtained.

Based on the experiment, the following preferable culture conditions were determined in the P1 phase:

After passage 1, the preferable initial cell density is 2 x 10³ - 1.5 x 10⁴ cells/ cm², a more preferable is 5 x 10³ - 1 x 10⁴ cells/ cm², and the highly preferable is about 6-8 x 10⁴ cells/ cm² or about 7 x 10³ cells/cm².

The cells are cultured preferably until 70% confluency, highly preferably until 80% confluency, and even more preferably until at least or about 90% confluency.

P1 cultures reach 90% confluency typically in 7-15 days, preferably in 8-10 or 8-9 days; after that, the cells are frozen.

The time of the second passage, particularly at the given initial cell number, for canine MSCs, is 5-20 days, preferably 7-18 days, highly preferably 9-15 days.

For culturing, highly preferably culture vessels, e.g. petri dishes are used. The surface of the culture vessel is preferably 50-300 cm² or 100-200 cm², highly preferably 130-170 cm².

The growth of the MSCs is influenced by individual differences in the donors; thus, experiments were performed to determine the quality of the samples to be frozen.

### Analysis of the effect of resupplying fresh medium during the culturing of the P1 cell culture

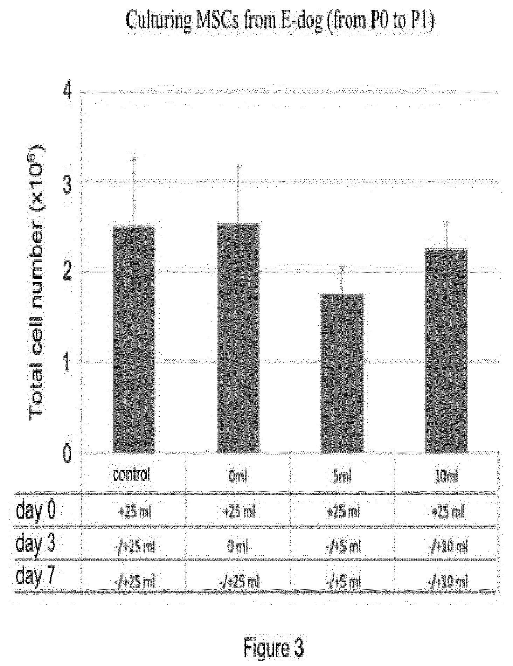

P1 cells derived from the dog designated Edog were cultured in 145 cm² petri dishes in 25 ml medium under four different conditions (see Figure 3). 10⁶ cells were plated in 25 ml on day 0. On days 3 and 7, the medium was changed according to the combinations shown in the figure (e.g. -/+ 25 ml indicates that the medium was totally removed from the cells and 25 ml fresh medium was added; -/+ 5 ml and -/+10ml indicate that, of the 25 ml medium, only 5 ml and 10 ml were removed, respectively, then the medium remaining on the cells was supplemented with 5 ml and 10 ml fresh medium, respectively). Cells were counted on day 9.

We found that it is preferable to change the total amount of the medium at least once during culturing. It is possible but not necessary to change the medium twice, e.g. it was sufficient to change the medium to fresh one after one week in the experiment.

### The effect of serum during isolation and culturing

Experiments were performed to determine whether the presence of serum, particularly that of horse serum, is beneficial in the different phases, e.g. during the culturing of cells freshly isolated from adipose tissue, during passage 1 and passage 2; in other words, e.g. before passage 1, after passage 1 but before passage 2, and preferably after passage 2.

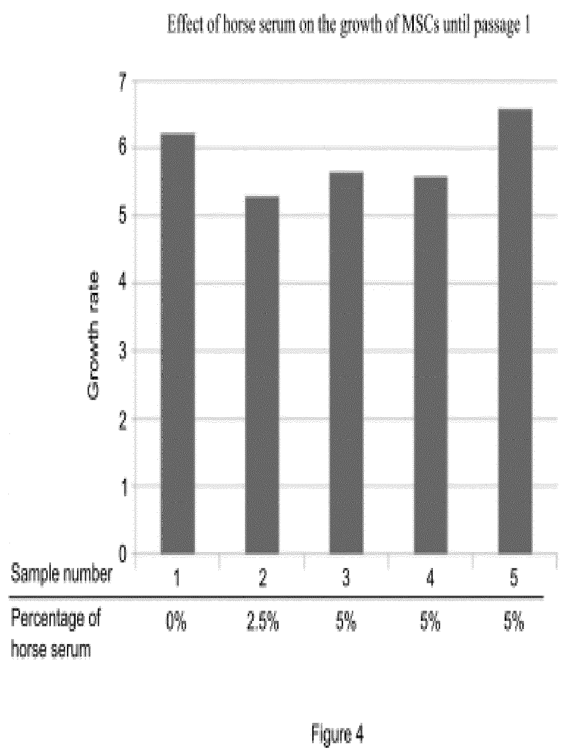

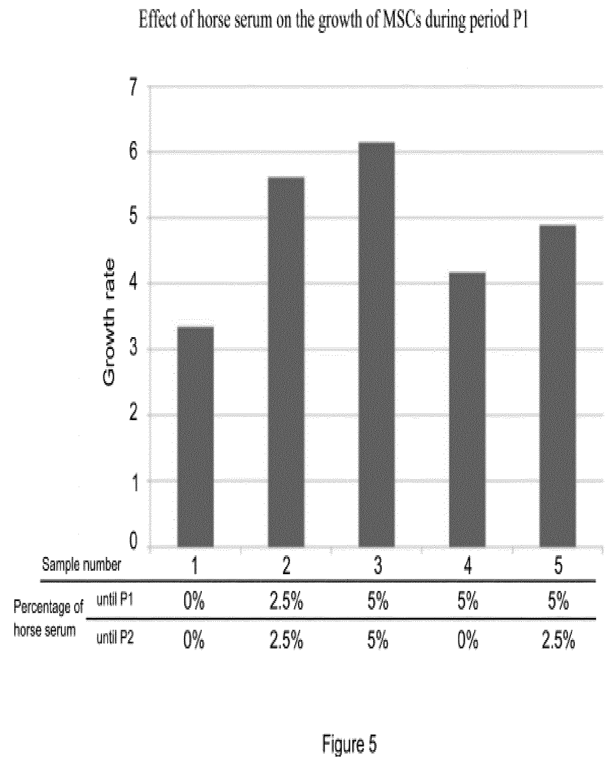

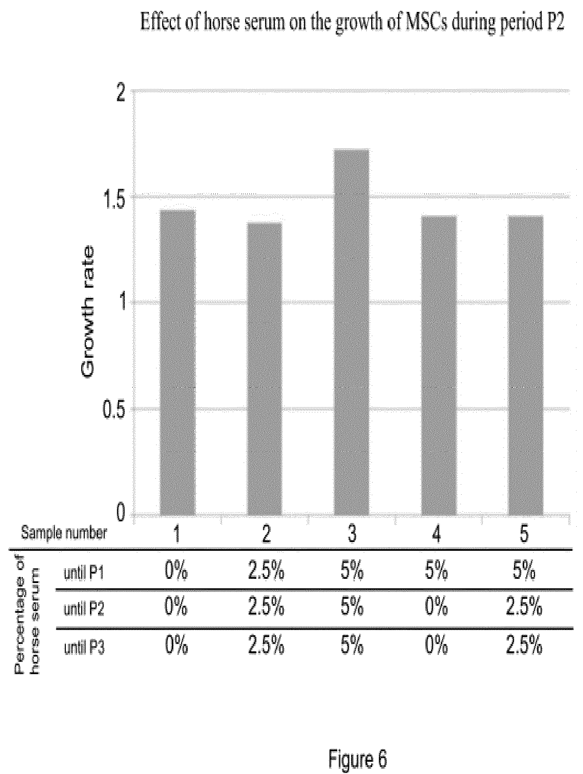

The results of these experiments are presented in Figures 4, 5, and 6.

Based on the experiments, we concluded that P0 cells do not require the presence of horse serum, and in P1 and P1 cultures 2.5% is sufficient instead of 5%. Though there is a slight increase in growth in the presence of 5% horse serum, the choice is to be contemplated.

The fact that the presence of horse serum is not required in the initial P0 culture can be explained by the presence of several other cell types at this time (white blood cells, stroma cells, etc.) besides the MSCs, which may provide the factors necessary for the survival of MSCs.

For the skilled person, it is evident from the above that other medium supplements that provide the necessary factors for the survival of MSCs, e.g. serum can be used instead of horse serum.

### The effects of freezing and thawing conditions on the viability of MSCs

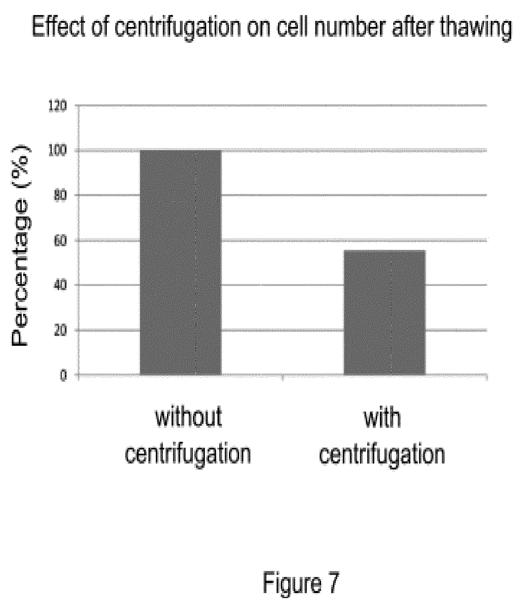

- According to a previous protocol, freezing was performed in ice-cold FCS+DMSO medium in a thermally isolated container.
- Preferably, freezing is performed for example in an isopropanol-filled freezing container (the result can be tested after thawing).
- The conditions of thawing were also tested (Figure 7). Based on the experiment, we determined the following preferable culture conditions after P2 (second passage). During thawing, instead of diluting and centrifuging the 10% DMSO-containing FCS freezing medium, cells are pipetted without centrifugation into the storage medium, preferably into complete medium, and transferred into the culturing vessel. Cell loss due to centrifugation can thus be avoided.

### Preparation of the cells for therapy

- For the treatment of a leg with elbow dysplasia, MSCs from at least two donors (10⁷ cells from each, frozen preferably during passage 1 and more preferably during passage 2) are thawed. Cells are diluted in 25 ml tissue culture medium (see MSC isolation protocol), and 1.5x10⁶ cells are plated into a 15 cm petri dish (10⁴ cells/cm²).
- The cells are grown for 3 days
- On the 3^{rd} day cells are detached from the bottom of the petri dishes with trypsin.
- The cells are centrifuged (see MSC isolation protocol), resuspended in PBS and counted.
- The cells derived from the two donors are mixed, preferably in a ratio of 1:1.
- Following centrifugation, the supernatant is discarded, and 0.5% hyaluronic acid is added to the cell pellet.
- For the treatment of one leg, 2x10⁷ MSCs are suspended in 2ml 0.5% hyaluronic acid, sucked into a syringe which is closed with a stopper. The syringe is placed into a sealable bag and placed into a paper box. Transportation is performed in a polysterene box with cooling (cooler block).
- Transplantation should be performed preferably within 24 hours. 85% of the cells can be recovered from the syringe.

It should be noted here that according to a previous protocol the cells were frozen at an earlier stage and cultured for 9-13 days after thawing with at least one, preferably two passages. Thus, previously, veterinarians had to order the MSCs 2 weeks in advance; now 4 days is enough.

### Analysis of cells prepared for therapy, stored in hyaluronic acid.

The viability of cells was examined in the condition they were in when prepared for therapy according to the example above.

We analysed the cells immediately after a 24-hour 0.5% hyaluronic acid (HA) treatment at room temperature or at 4°C in a syringe; that is, we determined the ratio of living and dead cells. Both samples contained 75% living and 25% dead cells.

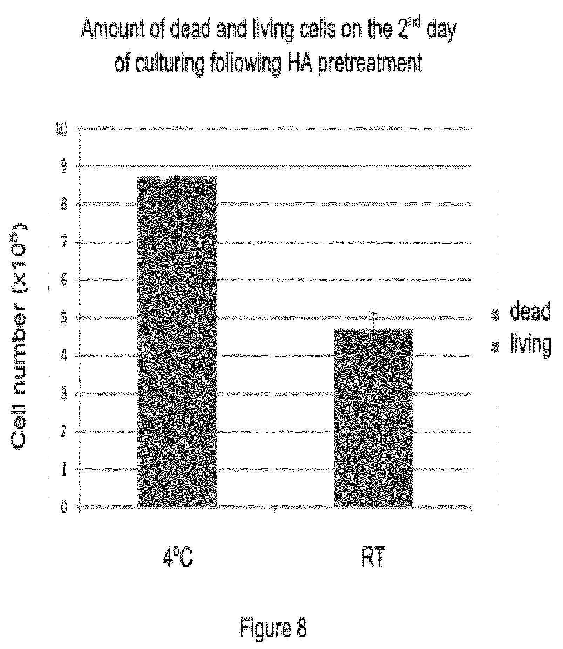

The cells were then further cultured for 2 days in tissue culture and counted again (Figure 8) to determine the number and ratio of living and dead cells following HA treatment. The figure demonstrates that the ratio of living cells following 2 days of culturing was significantly higher in the cells that were previously kept at 4°C in 0.5% hyaluronic acid.

Thus, we found that although the extent of cell death in HA is the same in the time period modelling storage and transport, irrespective of whether storage was performed at room temperature or at 4°C, during further culturing cells treated at 4°C are much more viable. Thus it is advisable to perform transportation in HA at low temperature (e.g. 4 C).

### Example 2

### Canine MSC isolation, propagation in tissue culture, freezing, and storage

### PSlDonor: dog

age: under 2 years
weight: ideal
volume of adipose tissue: 5-20 ml
localization of adipose tissue: abdominal

### Materials and methods

beaker, 70% alcohol
tissue paper
sterile forceps
sterile petri dish
2 sterile scissors or scalpels
2 sterile spoons
50 ml sterile plastic centrifuge tubes
500 ml PBS (phosphate buffered physiological saline)
collagenase: Sigma, C5138;
DMEM/F12 medium (Life Technologies 31330095)
FCS (fetal calf serum) (Life Technologies 10500064)
Horse serum (Life Technologies, 26050088)
Hank's solution (Life Technologies, 14175053)
PBS pH 7.2 (Life Technologies, 20012019)
Trypsin-EDTA 0.05% (Life Technologies, 25300062)
L-Glutamine 200 mM (Life Technologies, 25030024)
Penicillin-Streptomycin (Pen/Strep) (10,000 U/ml); (Life Technologies,15140122)
syringe 20 ml
syringe filter
sterile needle
shaker, at 37°C

Cell counting solution: Distilled water Trypan blue (DT) solution (800µl trypan blue and 200 µl distilled water). The hypotonic solution causes lysis of the red blood cells to facilitate cell number determination of the primer isolate.

### Collection and processing of adipose tissue

Canine adipose tissue is aseptically removed during the ovariectomy of healthy female dogs. This step is typically performed by the veterinarian.

The adipose tissue is transported from the veterinarian to the laboratory: the adipose tissue is placed into 25 ml 4°C cold Hank's solution in a 50 ml sterile plastic tube. Transport is performed in ice. Adipose tissue has to be processed preferably within 24 hours; cell viability and yield are not affected within this period of time.

The course of processing:
- adipose tissue is shaken to cleanse it from blood,
- the weight of the adipose tissue is determined,
- the tissue is mushed using scissors and forceps or two scissors,
- the mushed tissue is transferred to a 50 ml centrifuge tube,
   ∘ room temperature PBS is added to a volume of 50 ml,
   ∘ the volume of the fat is determined,
   ∘ centrifugation: 8 minutes, 600 rpm, without brakes, at room temperature,
   ∘ meanwhile, the collagenase is dispensed,
- after centrifugation, the uppermost layer of fat/oil is carefully sucked off and discarded,
- the upper layer of fat is spooned into a clean 50 ml centrifuge tube and the procedure above is repeated.

### Collection of cells by dissociation with collagenase

- Preparation of collagenase solution:
   ∘ 0.2 % collagenase solution is prepared in Hank's buffer (the amount depends on the quantity of adipose tissue to be treated)
   o the collagenase powder is dissolved by vortexing, the solution is pipetted into a 50 ml centrifuge tube and filled to the appropriate volume
   o the solution is sterilized by filtration into a new 50 ml centrifuge tube
   o the adipose tissue is transferred to a 50 ml centrifuge tube and an equal volume of 0.2 % collagenase solution is added (for example: 15 ml fat + 30 mg collagenase in 15 ml Hank's buffer)
- the sample thus prepared is shaken at 250 rpm at 37°C for 30 minutes on a platform shaker
- following digestion, it is filled to 50 ml with Hank's buffer and centrifuged (1200 rpm, 10 minutes, without cooling)
   o the supernatant is removed and discarded
   ∘ 50 ml Hank's buffer is added to the cell pellet, it is shaken, and centrifuged as described before
   o the cell pellet is suspended in 5 ml culture tissue medium (DMEM/F12, 2mM Glutamine, Pen/strep, 10% FCS 5% horse serum) (complete medium)
   o cell number is determined (50 µl cells+450 µl DT)

### Culturing: P0-Px refers to the number of passages

- 1.5 x 10⁶ cells in 25 ml volume are plated in a 15 cm petri dish (10⁴ cell/cm²). Tissue culture medium: DMEM/F12, 2mM Glutamine, Pen/strep, 10% FCS 5% horse serum (complete medium). P0 cells reach 90% confluency in about 5-7 days.
- On day 3, the culture is washed with 10 ml PBS to remove unattached cells, then culturing is continued in complete medium. The medium is changed every 4-7 days (according to a less preferable version every 2-3 days).
- Passage: the propagated P0 cells are detached from the bottom of the culture vessel using 0.05% trypsin (Table 1) and are plated again at 1.5 x 10⁶ cells/petri dish (10⁴ cells/cm²). The medium is changed on the cultures every 4-7 days (according to a less preferable version every 2-3 days).
- P1 cells reach 90% confluency in about 9-15 days, then the cells are detached with trypsin as above and taken through another culturing cycle similarly to the procedure described before.
- P2 cells are detached with trypsin and frozen.

The composition of trypsin is given in Table 1 below:

**Table 1**

| | | |
|---|---|---|
| Disodium hydrogen phosphate (Na₂HPO₄-7H₂O | Na₂HPO₄.7H₂O | 0.3 mM |

| **Other components** | | |
|---|---|---|
| D-glucose (Dextrose) | Dextrose | 5.6 mM |
| EDTA 4Na 2H₂O | EDTA 4Na 2H₂O | 0.9 mM |
| Phenol Red | Phenol Red | 0.03 mM |
| Trypsin | Trypsin | 0.1 mM |

The composition of the complete medium is given in Table 2 below:

**Table 2**

| Complete medium | | |
|---|---|---|
| DMEM / F 12 | | |
| FCS | 10% | |
| Horse serum | 5% | |
| Glutamine | 2mM | |
| Pen/strep mixture | 50 U/ml | |

### Freezing

- P2 cells are detached from the bottom of the culture vessel with 0.05% trypsin, and trypsin is neutralized with Hank's solution containing 10 % FCS (Fetal Calf Serum)
- Cells are collected in a centrifuge tube and sedimented by centrifuging
- Cell pellet is suspended in room temperature FCS
- Cell concentration is adjusted to 10⁷ cells/900 µl with FCS after cell counting
- 100 µl dimetil-szulfoxidot (DMSO) is added to the 900 µl cell suspension (in FCS) while vortexing (freezing solution: 10% DMSO in FCS)
- Cells are dispensed into freezing tubes (10⁷ cell/1ml freezing solution /tube). For therapy, to mix MSCs from two different donors, two tubes of cells have to be thawed and mixed.
- The tubes are placed in Nalgene® 5100-0001 Cryol°C "Mr. Frosty" freezing containers, filled with room temperature isopropanol and placed in -80°C for 24 hours.

### Miscellaneous

- 3 tubes of cells (3 x 10⁶ cells/tube) are archived for further analyses for at least one year
- An aliquot of the culture prepared for freezing is cultured further, and its bacteriological analysis is performed. At present, this involves testing for Mycoplasma.

### Example 3

### Equine MSC isolation, propagation in tissue culture, freezing, and storage

Species: horse; volume of adipose tissue: 5-20 ml; localization of adipose tissue: inguinal.

Equine MSC isolation, propagation in tissue culture, freezing, and storage is performed the same way as described for canine MSCs in example 1 with the difference that aseptic removal of equine adipose tissue is performed in narcosis from the inguinal area of healthy horses.

Our observations were in essence identical with the observations made with canine MSCs in example 1 with the difference that P1 cells reach 90% confluency in 30 days. Then, just like in the case of canine P1 cells, cells are detached with trypsin after reaching 90% confluency and frozen.

### Example 4

### Production of a preparation for therapeutic use

- For the treatment of a leg with elbow dysplasia, MSCs from two donors are thawed (10⁷ cells from each). Cells are diluted in 25 ml tissue culture medium (see MSC isolation protocol), and 1.5 x 10⁶ cells are plated in a 15 cm petri dish (10⁴ cells/cm²).
- The cells are grown for 3 days.
- On the 3^{rd} day, cells are detached from the bottom of the petri dishes with trypsin.
- The cells are centrifuged (according to the MSC isolation protocol in examples 1 and 2), resuspended in PBS and counted.
- The cells derived from the two donors are mixed, preferably in a ratio of 1:1.
- Following centrifugation, the supernatant is discarded, and 0.5% hyaluronic acid is added to the cell pellet.
- For the treatment of one leg, 2 x 10⁷ MSCs are suspended in 2 ml 0.5% hyaluronic acid thus producing the preparation for therapeutic treatment. The preparation is sucked into a syringe which is closed with a stopper. The syringe is packed, e.g. into a sealable bag and paper box. The preparation thus packed is transported in a polysterene box with cooling (cooler block).
- The preparation is used for transplantation within 24 hours of adding hyaluronic acid.

### Example 5

### Treatment of dogs

The dogs were given pain relief medication (NSAID, 2-4 mg Carprophen/kg body weight) for one week prior to treatment and were prescribed restful regimen after the treatment.

The treatment is an ambulatory one, involving a 15-minute intervention by the veterinarian and is considered a mild treatment performed in narcosis. During treatment, 1 ml synovial fluid is sucked out of the joint with a syringe and an equal volume of MSCs suspended in 0.5% isotonic hyaluronic acid (Viscoseal TRB CHEMEDICA UK, manufacturer: TRB CHEMEDICA AG. Haar/München, DE) is injected back directly into the joint. (For elbow and hip dysplasia, the stem cells are injected directly into the elbow and the hip, respectively.)

Restful regimen is prescribed for after the treatment and monitoring is performed for at least 3-12 months.

If necessary, the following medications can be applied:
- Premedication: Phentanyl injection + Midazolam injection+Ketamin injection i.v. -intubation: Propofol injection
- Narcosis: Isofluran+ Salsol in infusion: Phentanyl+Ketamin
- Local supplementation: Lidocain injection; Lidocain should be administered intraarticularly as well, in case of arthroscopy
- Postoperative pain relief medication: for severe pain: Tramadol pill; for mild or moderate pain Meloxicam pill.

### Example 6

### Evaluation of the result of the treatment

Evaluation of the approved veterinary symptoms is based on the observations of the veterinarian and the owner: lameness in walking and trotting, pain to touch, pressure, range of motion, functional abilities, inflammation/need for pain relief medication, individual scoring of asymptomatic period from 1 to 5. Statistic evaluation of the results is performed with the Friedman test. During post hoc analysis, Tukey's method is used for multiple comparison. Scoring is performed with the modified Black LL method (Black LL et al. 2007 Vet Therap 8:272-84) according to the table attached (Table 3):

**Table 3**

| | Cannot be detected | Intermittent | Persistent | Persistent - unable to bear weight | Can only walk with help | Unable to walk |
|---|---|---|---|---|---|---|
| Lameness in walking | 1 | 2 | 3 | 4 | 5 | 6 |
| Lameness in trotting | 1 | 2 | 3 | 4 | 5 | 6 |

| | No pain | | Mild pain - tries to pull up its leg | | Severe pain - pulls its leg back immediately | |
|---|---|---|---|---|---|---|
| Pain - to touch, pressure, movement | 1 | | 2 | | 3 | |

| | No limitation | Pain at the end point only | | Pain before the end point | | Pain to movement of the joint |
|---|---|---|---|---|---|---|
| Functional abilities | 1 | 2 | | 3 | | 4 |

| | Not required | | Intermittently required | | Constant pain relief required | |
|---|---|---|---|---|---|---|
| Requirement of pain relief medication | 1 | | 2 | | 3 | |

| | 12 months | 9 months | 6 months | | 3 months | 1 month |
|---|---|---|---|---|---|---|
| Asymptomatic period | 1 | 2 | 3 | | 4 | 5 |

We obtained the following results after performing the surgery and the evaluation with the methods described above.

### Example 6.1

Social security number: bono20110603
Name, breed, sex, weight: Bonó, Golden Retriever, male, 30 kg
Date of birth: 03/06/2011
Diagnosis: Severe and mild elbow arthrosis of the left and right elbow, respectively.
Date of surgery: Left: 12/01/2012 (conventional); right: arthroscopic, 28/09/2012
MSC transplantation: both elbows 22/05/2013 MSC code: D1301.2 (1.3 x 10⁷)
Left/Right

| | Before treatment | 1 week after | 2 weeks after | 3 weeks after | 4 weeks after | 6 weeks after | 3 months after | 6 months after | 9 months after | 2 years after |
|---|---|---|---|---|---|---|---|---|---|---|
| Lameness in walking | 2/1 | 2/1 | 1 | 1 | 1 | 1 | 1 | 1 | 1 | 1 |
| | | | | | | | | | | |
| Lameness in trotting | 2/1 | 2/1 | 1 | 1 | 1 | 1 | 1 | 1 | 1 | 1 |
| | | | | | | | | | | |
| Pain to touch, pressure, movement | 2/1 | 2/1 | 1 | 1 | 1 | 1 | 1 | 1 | 1 | 1 |
| | | | | | | | | | | |
| Free movement | 2/1 | 1/1 | 1 | 1 | 1 | 1 | 1 | 1 | 1 | 1 |
| | | | | | | | | | | |
| Functional abilities | 3/1 | 2/1 | 2/1 | 2/1 | 2/1 | 2/1 | 2/1 | 2/1 | 2/1 | 2 |
| | | | | | | | | | | |
| Pain relief medication | 2 | 1 | 1 | 1 | 1 | 1 | 1 | 1 | 1 | 1 |

| | | | | | | | | | | |
|---|---|---|---|---|---|---|---|---|---|---|
| Note: the left knee is still stiff and thick | | | | | | | | | | |

### Example 6.2

Social security number: kifli20131124
Name, breed, sex, weight: Kifli, Golden Retriever, male, 27 kg
Date of birth: 24/11/2013
Diagnosis: bilateral ED, fragmented processus coronoideus (FCP), cartilage detachment, arthrosis
Date of surgery: left: 21/05/2014 arthroscopic, proximal ulnar osteotomy; right: 25/07/2014
MSC transplantation: 27/08/2014; MSC code: D1403.3 (0.9 x 10⁷)

| | Before treatment | 1 week after | 2 weeks after | 3 weeks after | 4 weeks after | 6 weeks after | 3 months after | 6 months after | 9 months after | 1 year after |
|---|---|---|---|---|---|---|---|---|---|---|
| Lameness in walking | 2 | 2 | 1 | 1 | 1 | 1 | 2 | 1 | 1 | 1 |
| | | | | | | | | | | |
| Lameness in trotting | 2 | 2 | 2 | 1 | 1 | 1 | 2 | 1 | 1 | 1 |
| | | | | | | | | | | |
| Pain to touch, pressure, movement | 2 | 2 | 1 | 1 | 1 | 1 | 1 | 1 | 1 | 1 |
| | | | | | | | | | | |
| Free movement | 2 | 2 | 1 | 1 | 1 | 1 | 1 | 1 | 1 | 1 |
| | | | | | | | | | | |
| Functional abilities | 2 | 2 | 2 | 1 | 1 | 1 | 2 | 1 | 1 | 1 |
| | | | | | | | | | | |
| Pain relief medication | 1 | 2 | 1 | 1 | 1 | 1 | 1 | 1 | 1 | 1 |

| | | | | | | | | | | |
|---|---|---|---|---|---|---|---|---|---|---|
| Note: 3^{rd} month: lameness is less frequent 5^{th} month: occasional limping when tired during walk | | | | | | | | | | |

### Example 6.3

Social security number: chili20101224
Name, breed, sex, weight: Chili, Dogue de Bordeoux/Amstaff, male, 48 kg
Date of birth: 24/12/2010
Diagnosis: bilateral severe ED, bilateral fragmented processus coronoideus (cartilage detachment, arthrosis)
Date of surgery: left: 21/05/2014 arthroscopy, proximal ulnar osteotomy; right: 25/07/2014
MSC transplantation: 27/08/2014; MSC code: D1403.3 (0.9 x 10⁷)

| | Before treatment | 1 week after | 2 week afters | 3 week afters | 4 week afters | 6 week afters | 3 months after | 6 months after | 9 months after | 1 year after |
|---|---|---|---|---|---|---|---|---|---|---|
| Lameness in walking | 3 | 3 | 3 | 2 | 2 | 2 | 3 | 1 | 1 | 1 |
| | | | | | | | | | | |
| Lameness in trotting | 3 | 3 | 3 | 3 | 2 | 2 | 3 | 1 | 1 | 1 |
| | | | | | | | | | | |
| Pain to touch, pressure, movement | 2 | 3 | 3 | 2 | 2 | 1 | 2 | 1 | 1 | 1 |
| | | | | | | | | | | |
| Free movement | 2 | 2 | 2 | 2 | 2 | 1 | 4 | 1 | 1 | 1 |
| | | | | | | | | | | |
| Functional abilities | 2-3 | 2-3 | 2 | 2 | 2 | 2 | 3 | 1 | 1 | 1 |
| | | | | | | | | | | |
| Pain relief medication | 2 | 2 | 1 | 1 | 1 | 1 | 2 | 1 | 1 | 1 |

| | | | | | | | | | | |
|---|---|---|---|---|---|---|---|---|---|---|
| Note: 2^{nd} month: infrequent lameness 3^{rd} month: inflammation of the left shoulder joint, treatment, lameness again 5^{th} month: no lameness, normal activity | | | | | | | | | | |

### Example 6.4

Social security number: bukfenc20140120
Name, breed, sex, weight: Bukfenc, Golden Retriever, male, 28 kg
Date of birth: 20/01/2014
Diagnosis: unilateral severe ED, severe medial processus coronoideus detachment on right leg
Date of surgery: 26/03/2014 arthroscopy
MSC transplantation: 27/08/2014; MSC code: D1403.3 (0.9 x 10⁷)

| | Before treatment | 1 week after | 2 weeks after | 3 weeks after | 4 weeks after | 6 weeks after | 3 months after | 6 months after | 9 months after | 1 year after |
|---|---|---|---|---|---|---|---|---|---|---|
| Lameness in walking | 3 | 4 | 3 | 3 | 3 | 2 | 2 | 1 | 1 | 1 |
| | | | | | | | | | | |
| Lameness in trotting | 3 | 3 | 3 | 3 | 3 | 3 | 2 | 1 | 1 | 1 |
| | | | | | | | | | | |
| Pain to touch, pressure, movement | 2 | 3 | 2 | 2 | 2 | 2 | 1 | 1 | 1 | 1 |
| | | | | | | | | | | |
| Free movement | 2 | 3 | 2 | 2 | 2 | 2 | 1 | 1 | 1 | 1 |
| | | | | | | | | | | |
| Functional abilities | 2-3 | 3 | 2-3 | 2 | 2 | 1 | 1 | 1 | 1 | 1 |
| | | | | | | | | | | |
| Pain relief medication | 1 | 2 | 1 | 1 | 1 | 1 | 1 | 1 | 1 | 1 |

| | | | | | | | | | | |
|---|---|---|---|---|---|---|---|---|---|---|
| Note: 2^{nd} month: normal activity, the dog slightly reduces load on the affected leg 3^{rd} month: lame in cold, normal activity, the dog slightly reduces load, mildly lame after rest 5^{th} month: as in the 3^{rd} month, normal activity, sensitive to weather fronts, limps only when overburdened | | | | | | | | | | |

### Example 6.5

Social security number: lena20140625
Name, breed, sex, weight: Léna, Rottweiler, female, 34 kg
Date of birth: 25/06/2014
Diagnosis: left ankle OCD (severe)
Date of surgery: 20/06/2014; implant removal: 20/10/2014
MSC transplantation: 26/11/2014; MSC code: D14042+D1406.2 (mixed in a ratio of 3:1, in a total of 10⁷ cells/leg)

| | Before treatment | 1 week after | 2 weeks after | 3 weeks after | 4 weeks after | 2 months after | 3 months after | 6 months after | 9 months after | 1 year after |
|---|---|---|---|---|---|---|---|---|---|---|
| Lameness in walking | 1 | | | 1 | | 1 | 1 | 1 | 1 | 1 |
| | | | | | | | | | | |
| Lameness in trotting | 1 | | | 1 | | 1 | 1-2 | 1 | 1 | 1 |
| | | | | | | | | | | |
| Pain to touch, pressure, movement | 1 | | | 1 | | 1 | 1 | 1 | 1 | 1 |
| | | | | | | | | | | |
| Free movement | 1 | | | 1 | | 1 | 1 | 1 | 1 | 1 |
| | | | | | | | | | | |
| Functional abilities | 1 | | | 1 | | 1 | 1 | 1 | 1 | 1 |
| | | | | | | | | | | |
| Pain relief medication | 1 | | | 1 | | 1 | 1 | 1 | 1 | 1 |

| | | | | | | | | | | |
|---|---|---|---|---|---|---|---|---|---|---|
| Note: regularly attends physiotherapy, its leg is more sensitive after transplantation, but it is not lame. She is a dog with extreme exercise needs. 3^{rd} month: mild lameness, infrequently | | | | | | | | | | |

### Example 6.6

Social security number: molly20060801
Name, breed, sex, weight: Molly, Chow Chow, female, 32 kg
Date of birth: 01/08/2006
Diagnosis: both elbows ED arthroscopy
Date of surgery: Left leg: 09/07/2014 Right leg: 03/09/2014
MSC transplantation: 26/11/2014 MSC code: D1404.2+D1406.2 (mixed in a ratio of 3:1, in a total of 10⁷ cells/leg)

| | Before treatment | 1 week after | 2 weeks after | 3 weeks after | 4 weeks after | 2 months after | 3 months after | 6 months after | 9 months after | 1 year after |
|---|---|---|---|---|---|---|---|---|---|---|
| Lameness in walking | 3 | | | 1 | 1 | 1 | 1 | 1 | 1 | 1 |
| | | | | | | | | | | |
| Lameness in trotting | 3 | | | 1 | 1 | 1 | 1 | 1 | 1 | 1 |
| | | | | | | | | | | |
| Pain to touch, pressure, movement | 1 | | | 1 | 1 | 1 | 1 | 1 | 1 | 1 |
| | | | | | | | | | | |
| Free movement | | | | 1 | 1 | 1 | 1 | 1 | 1 | 1 |
| | | | | | | | | | | |
| Functional abilities | | | | 1 | 1 | 1 | 1 | 1 | 1 | 1 |
| | | | | | | | | | | |
| Pain relief medication | | | | 1 | 1 | 1 | 1 | 1 | 1 | 1 |

| | | | | | | | | | | |
|---|---|---|---|---|---|---|---|---|---|---|
| Note: Received HA one and a half months before MSCs, but it was effective for one month 3^{rd} week: no walks with the dog 4^{th} week: walks on leash, spectacular improvement 2^{nd} month: displays limping in cold after getting up 3^{rd} month: a slight limp only after excessive walk. | | | | | | | | | | |

Green lipped mussel, curcuma.

### Example 6.7

Social security number: donci20110625
Name, breed, sex, weight: Dönci, Labrador Retriever, male, 35 kg
Date of birth: 25/06/2011
Diagnosis: bilateral ED
Date of surgery: arthroscopy left leg: 25/07/2014; right leg: 03/09/2014
MSC transplantation: 03/12/2014; MSC code: D1404.2 (10⁷)

| | Before treatment | 1 week after | 2 weeks after | 3 weeks after | 4 weeks after | 2 months after | 3 months after | 6 months after | 9 months after | 1 year after |
|---|---|---|---|---|---|---|---|---|---|---|
| Lameness in walking | 1 | | | 1 | 1 | 1 | 1 | 1 | 1 | 1 |
| | | | | | | | | | | |
| Lameness in trotting | 1 | | | 1 | 1 | 1 | 1 | 1 | 1 | 1 |
| | | | | | | | | | | |
| Pain to touch, pressure, movement | 1 | | | 1 | 1 | 1 | 1 | 1 | 1 | 1 |
| | | | | | | | | | | |
| Free movement | 1 | | | 1 | 1 | 1 | 1 | 1 | 1 | 1 |
| | | | | | | | | | | |
| Functional abilities | 1 | | | 1 | 1 | 1 | 1 | 1 | 1 | 1 |
| | | | | | | | | | | |
| Pain relief medication | 1 | | | 1 | 1 | 1 | 1 | 1 | 1 | 1 |

| | | | | | | | | | | |
|---|---|---|---|---|---|---|---|---|---|---|
| Note: Previously, infrequent bad limping; after surgeries, intermittent bad limping. This ceased totally; there is no limping after the treatment at all, without leash 6^{th} month: totally asymptomatic 9^{th} month: perfect 11^{th} month: perfect | | | | | | | | | | |

### Example 6.8

Social security number: vadoc20130629
Name, breed, sex, weight: Vadóc, Hungarian Vizsla, female, 21 kg
Date of birth: 29/06/2013
Diagnosis: ED on both legs, coronoidea detachment in both joints (verified by X-ray)
Date of surgery: Left: 03/09/2014 Right: 29/09/2014
MSC transplantation: left: 01/10/2014; right: 27/10/2014;
MSC code: left leg D1301.3 (2 x 10⁷), right leg D1301.3 (1.2 x 10⁷)
Left/Right

| | Before treatment | 1 week after | 2 weeks after | 3 weeks after | 4 weeks after | 6 weeks after | 3 months after | 6 months after | 9 months after | 1 year after |
|---|---|---|---|---|---|---|---|---|---|---|
| Lameness in walking | 2 | 2/3 | 1/2 | 1 | 1 | 1 | 1 | 1 | 1 | 1 |
| | | | | | | | | | | |
| Lameness in trotting | 2 | 2/4 | 1/2 | 1 | 1 | 1 | 1 | 1 | 1 | 1 |
| | | | | | | | | | | |
| Pain to touch, pressure, movement | 2 | 2/3 | 1/2 | 1 | 1 | 1 | 1 | 1 | 1 | 1 |
| | | | | | | | | | | |
| Free movement | 2/3 | 1/4 | 1/2 | 1 | 1 | 1 | 1 | 1 | 1 | 1 |
| | | | | | | | | | | |
| Functional abilities | 3 | 2/4 | 2/3 | 2 | 2 | 2 | 2 | 2 | 2 | 1 |
| | | | | | | | | | | |
| Pain relief medication | 2 | 1 | 1 | 1 | 1 | 1 | 1 | 1 | 1 | 1 |

| | | | | | | | | | | |
|---|---|---|---|---|---|---|---|---|---|---|
| Note: Hind legs are spread slightly in walk, runs well, active, but cannot keep up with its sibling in running. | | | | | | | | | | |

### Example 6.9

Social security number: mira20040831
Name, breed, sex, weight: Mira, Belgian Shepherd, female, 30 kg
Date of birth: 31/08/2004
Diagnosis: knee OCD
Date of surgery: 2010
MSC transplantation: 03/12/2014; MSC code: D1404.2 (10⁷)

| | Before treatment | 1 week after | 2 weeks after | 3 weeks after | 5 weeks after | 2 months after | 3 months after | 6 months after | 9 months after | 1 year after |
|---|---|---|---|---|---|---|---|---|---|---|
| Lameness in walking | 1 | | 1 | | 1 | 1 | 1 | 1 | 1 | 1 |
| | | | | | | | | | | |
| Lameness in trotting | 1 | | 1 | | 1 | 1 | 1 | 1 | 1 | 1 |
| | | | | | | | | | | |
| Pain to touch, pressure, movement | 2 | | 2 | | 1 | 1 | 1 | 1 | 1 | 1 |
| | | | | | | | | | | |
| Free movement | 4 | | 4 | | 1 | 1 | 1 | 1 | 1 | 1 |
| | | | | | | | | | | |
| Functional abilities | 1 | | 1 | | 1 | 1 | 1 | 1 | 1 | 1 |
| | | | | | | | | | | |
| Pain relief medication | 3 | | 1 | | 1 | 1 | 1 | 1 | 1 | 1 |

| | | | | | | | | | | |
|---|---|---|---|---|---|---|---|---|---|---|
| Note: Constantly takes Trocoxil 2^{nd} week: gets up with difficulty, less lively, walks only. Did not get Trocoxil Has not been given medication for 2 months, active, no lameness. 3^{rd} month: does not take Trocoxil. A slight limp when the leg is overburdened. 6^{th} month: does not take Trocoxil. The dog is in good condition, active for its age. No limping. | | | | | | | | | | |

### Example 6.10

Social security number: carlos20111103
Name, breed, sex, weight: Carlos, Labrador Retriever, male, 40 kg
Date of birth: 03/11/2011
Diagnosis: ED, left elbow is worse. Limps since puppyhood
Date of surgery: 02/2014
MSC transplantation: 03/12/2014; MSC code: D1404.2 (10⁷)

| | Before treatment | 1 week after | 2 weeks after | 3 weeks after | 5 weeks after | 2 months after | 3 months after | 6 months after | 9 months after | 1 year after |
|---|---|---|---|---|---|---|---|---|---|---|
| Lameness in walking | 2 | | 1 | 1 | 1 | 1 | 1 | 1 | 1 | 1 |
| | | | | | | | | | | |
| Lameness in trotting | 1 | | 1 | 1 | 1 | 1 | 1 | 1 | 1 | 1 |
| | | | | | | | | | | |
| Pain to touch, pressure, movement | 1 | | 1 | 1 | 1 | 1 | 1 | 1 | 1 | 1 |
| | | | | | | | | | | |
| Free movement | 1 | | 1 | 1 | 1 | 1 | 1 | 1 | 1 | 1 |
| | | | | | | | | | | |
| Functional abilities | 3 | | 2 | 1 | 1 | 1 | 1 | 1 | 1 | 1 |
| | | | | | | | | | | |
| Pain relief medication | 1 | | 1 | 1 | 1 | 1 | 1 | 1 | 1 | 1 |

| | | | | | | | | | | |
|---|---|---|---|---|---|---|---|---|---|---|
| Note: 2^{nd} week: walk on leash 5^{th} week: runs without leash 2^{nd} month: very active, runs; if overtired, hobbles. Its leg does not bend fully. 3^{rd} month: a slight stiffness of the leg remains. 6^{th} month: lies down when tired, but does not limp even then! Everything is all right. | | | | | | | | | | |

### Example 6.11

Social security number: nanook20070316
Name, breed, sex, weight: Nanook, German Shepherd, male, 30 kg
Date of birth: 16/03/2007
Diagnosis: osteophytes in the hip joint
Date of surgery: there was no surgery
MSC transplantation: 03/12/2014 MSC code: D1404.2 (10⁷)

| | Before treatment | 1 week after | 2 weeks after | 3 weeks after | 5 weeks after | 2 months after | 3 months after | 6 months after | 9 months after | 1 year after |
|---|---|---|---|---|---|---|---|---|---|---|
| Lameness in walking | 2 | | 1 | 1 | 1 | 1 | 1 | 1 | 1 | 1 |
| | | | | | | | | | | |
| Lameness in trotting | 1 | | 1 | 1 | 1 | 1 | 1 | 1 | 1 | 1 |
| | | | | | | | | | | |
| Pain to touch, pressure, movement | 1 | | 1 | 1 | 1 | 1 | 1 | 1 | 1 | 1 |
| | | | | | | | | | | |
| Free movement | 1 | | 1 | 1 | 1 | 1 | 1 | 1 | 1 | 1 |
| | | | | | | | | | | |
| Functional abilities | 3 | | 1 | 1 | 1 | 1 | 1 | 1 | 1 | 1 |
| | | | | | | | | | | |
| Pain relief medication | 1 | | 1 | 1 | 1 | 1 | 1 | 1 | 1 | 1 |

| | | | | | | | | | | |
|---|---|---|---|---|---|---|---|---|---|---|
| Note: 2^{nd} week: walk on leash 5^{th} week: runs, active 2^{nd} month: hobbles during weather fronts 6^{th} month: still not lame; moreover, can jump hurdles. 9^{th} month: the same as the 6^{th} month. Runs, jumps a lot. In case of extreme weather changes, Meloxydil is given on that day. 11^{th} month: the same as the 6^{th} month. Runs, jumps a lot. In case of extreme weather changes, Meloxydil is given on that day. | | | | | | | | | | |

### Example 6.12

Social security number: dusty20130226
Name, breed, sex, weight: Dusty, Cane Corso, male, 51 kg
Date of birth: 26/02/2013
Diagnosis: unilateral ED, removal of the proc. anconeus, minimal cartilage degeneration on the surface of proc. coronoideus
Date of surgery: 28/11/2014, arthroscopy, removal of the isolated proc. anconeus
MSC transplantation: 17/12/2014 MSC code: D1404.2+D1406.2 (1:1, a total of 2 x 10⁷ cells/leg)

| | Before treatment | 1 week after | 2 weeks after | 3 weeks after | 5 weeks after | 2 months after | 3 months after | 6 months after | 9 months after | 1 year after |
|---|---|---|---|---|---|---|---|---|---|---|
| Lameness in walking | 3 | | | 2 | 1 | 1 | 1 | 1 | 2 | 3 |
| | | | | | | | | | | |
| Lameness in trotting | 2 | | | 2 | 1 | 1 | 1 | 1 | 2 | 3 |
| | | | | | | | | | | |
| Pain to touch, pressure, movement | 2 | | | 1 | 1 | 1 | 1 | 1 | 2 | 2 |
| | | | | | | | | | | |
| Free movement | 2 | | | 1 | 1 | 1 | 1 | 1 | 2 | 2 |
| | | | | | | | | | | |
| Functional abilities | 2 | | | 1 | 1 | 1 | 1 | 1 | 2 | 2 |
| | | | | | | | | | | |
| Pain relief medication | 2 | | | 1 | 1 | 1 | 1 | 1 | 2 | 3 |

| | | | | | | | | | | |
|---|---|---|---|---|---|---|---|---|---|---|
| Note: 3^{rd} week: lameness only after getting up, rarely afterwards 5^{th} week: runs a lot, a mild lameness after getting up. After the 2^{nd} month, lameness is even less visible after excessive exercise. Was given Rimadyl occasionally. 3^{rd} month: no lameness 6^{th} month: Still no lameness, and though the joint remained slightly stiff, it functions perfectly. | | | | | | | | | | |

### Example 6.13

Social security number: nestor20050701
Name, breed, sex, weight: Nestor, Labrador Retriever, male, 37.5 kg
Date of birth: 01/07/2005
Diagnosis: unilateral severe (Outerbridge-III) loss of cartilage in the area of coronoideus medialis, the radial head, and the condylus medialis; fragmented proc. coronoideus is not visible
Date of surgery: 11/11/2014 arthrosis art. cubiti dextri
MSC transplantation: 17/12/2014 MSC code: D1404.2+D1406.2 (1:1, in a total of 2 x 10⁷ cells/leg)

| | Before treatment | 1 week after | 3 weeks after | 5 weeks after | 2 months after | 3 months after | 6 months after | 9 months after | 1 year after |
|---|---|---|---|---|---|---|---|---|---|
| Lameness in walking | 3 | | 3 | 2 | 2 | 2 | 2 | 2 | |
| | | | | | | | | | |
| Lameness in trotting | 3 | | 3 | 2 | 2 | 2 | 2 | 2 | |
| | | | | | | | | | |
| Pain to touch, pressure, movement | 1 | | 1 | 1 | 1 | 1 | 2 | 1 | |
| | | | | | | | | | |
| Free movement | 4 | | 3 | 1 | 1 | 1 | 1 | 1 | |
| | | | | | | | | | |
| Functional abilities | 3 | | 3 | 1 | 1 | 1 | 1 | 1 | |
| | | | | | | | | | |
| Pain relief medication | 3 | | 2 | 1 | 1 | 1 | 2 | 2 | |

| | | | | | | | | | |
|---|---|---|---|---|---|---|---|---|---|
| Note: 5^{th} week: began to show improvement after the 4^{th} week. At the end of walks, hobbles on its leg. | | | | | | | | | |

### Example 6.14

Social security number: barney20130919
Name, breed, sex, weight: Barney, mixed breed, male, 23 kg
Date of birth: 19/09/2013
Diagnosis: left elbow outerbridge IV, fragmented, cartilage-less, the detached piece cannot be removed by arthroscopy, thus, subtotal coronoidectomy
Date of surgery: 26/11/2014 left elbow arthroscopy, renewal of the cartilage-less areas; the detached piece cannot be removed by arthroscopy; subtotal coronoidectomy via arthrotomy, removal of the detached piece.
MSC transplantation: 17/12/2014 Left leg. MSC code: D140.42+D1406.2 (1:1, in a total of 2 x 10⁷ cells/leg)

| | Before treatment | 1 week after | 2 weeks after | 3 weeks after | 5 weeks after | 2 months after | 3 months after | 6 months after | 9 months after | 1 year after |
|---|---|---|---|---|---|---|---|---|---|---|
| Lameness in walking | 3 | | | 1 | 1 | 1 | 1 | 1 | 1 | 1 |
| | | | | | | | | | | |
| Lameness in trotting | 3 | | | 2 | 2 | 2 | 1 | 1 | 1 | 1 |
| | | | | | | | | | | |
| Pain to touch, pressure, movement | 2 | | | 1 | 1 | 1 | 1 | 1 | 1 | 1 |
| | | | | | | | | | | |
| Free movement | 4 | | | 1 | 1 | 1 | 1 | 1 | 1 | 1 |
| | | | | | | | | | | |
| Functional abilities | 1 | | | 1 | 1 | 1 | 1 | 1 | 1 | 1 |
| | | | | | | | | | | |
| Pain relief medication | 2 | | | 2 | 2 | 1 | 1 | 1 | 1 | 1 |

| | | | | | | | | | | |
|---|---|---|---|---|---|---|---|---|---|---|
| Note: 5^{th} week: runs, mildly lame at the end. Runs without leash, he is not lame at the beginning, only slightly later. Seems to be better and better. 2^{nd} month: he is not lame basically; he limps on the affected leg only when runs excessively and is tired at the end of the day. 3^{rd} month: he has stem cells in his right leg as well. Uses both his front legs perfectly well. He is not lame. 6^{th} month: the dog is in perfect condition! | | | | | | | | | | |

### Example 6.15

Social security number: barney20130919
Name, breed, sex, weight: Barney, mixed breed, male, 23 kg
Date of birth: 19/09/2013
Diagnosis: Right elbow arthrosis without visible FCP.
Date of surgery: Did not undergo surgery.
MSC transplantation: 17/12/2014 Right leg. MSC code1: D1404.2+D1406.2 (1:1, in a total of 2x10⁷ cells/leg)

| | Before treatment | 1 week after | 2 weeks after | 3 weeks after | 4 weeks after | 6 weeks after | 3 months after | 6 months after | 9 months after | 1 year after |
|---|---|---|---|---|---|---|---|---|---|---|
| Lameness in walking | 1 | 1 | 1 | 1 | 1 | 1 | 1 | 1 | 1 | |
| | | | | | | | | | | |
| Lameness in trotting | 1 | 2 | 2 | 1 | 1 | 1 | 1 | 1 | 1 | |
| | | | | | | | | | | |
| Pain to touch, pressure, movement | 2 | 1 | 1 | 1 | 1 | 1 | 1 | 1 | 1 | |
| | | | | | | | | | | |
| Free movement | 2 | 1-2 | 1-2 | 1 | 1 | 1 | 1 | 1 | 1 | |
| | | | | | | | | | | |
| Functional abilities | 1 | 1-2 | 1-2 | 1 | 1 | 1 | 1 | 1 | 1 | |
| | | | | | | | | | | |
| Pain relief medication | 2 | 1 | 1 | 1 | 1 | 1 | 1 | 1 | 1 | |

| | | | | | | | | | | |
|---|---|---|---|---|---|---|---|---|---|---|
| **Note:** Already received cells in December in his left elbow joint. 1^{st} week: A mild lameness when he runs a little, but definitely uses his front legs better. 4^{th} week /3 and a half months since transplantation in the left leg: he already uses both his front legs well; he is not lame even after walks. (He has always been reducing load on his hind legs as a result of an earlier vertebral infection.) 9^{th} month: Both legs are in perfect condition! | | | | | | | | | | |

In sum, it can be stated that the treatment improved the condition of all of the treated animals remarkably, and though the symptoms did not fully cease in all cases, the quality of life of the dogs improved significantly.

### Example 7

### Assessment of the condition of the does after 12 months

### Patients

74 joints of 59 dogs were treated during this research. Dogs with lameness due to pain attributed to articular cartilage degeneration were included in the study. To obtain a homogeneous population with a sufficient number of cases, only cases involving elbow articular cartilage were included; out of the 74 cases, 19 were excluded due to other orthopaedic diseases, 8 due to the death of 7 dogs and 7 due to pain relief treatment, which influences pain sensation, thus, the follow-up of the MSC treatment. Thus, the efficiency of the MSC treatment was evaluated on 40 elbow joints altogether.

### Evaluation of the MSC treatment

The status of the treated dogs was evaluated by the owners on the basis of a questionnaire published by Black et. al. (Table 3). The extent of lameness observed during walk was used to describe the efficacy of the MSC therapy. Data were collected regularly, and results at the sixth, ninth, and twelfth months were demonstrated. Statistical analysis was done using non-parametric Friedman test and Tukey post hoc analysis. p<0,001

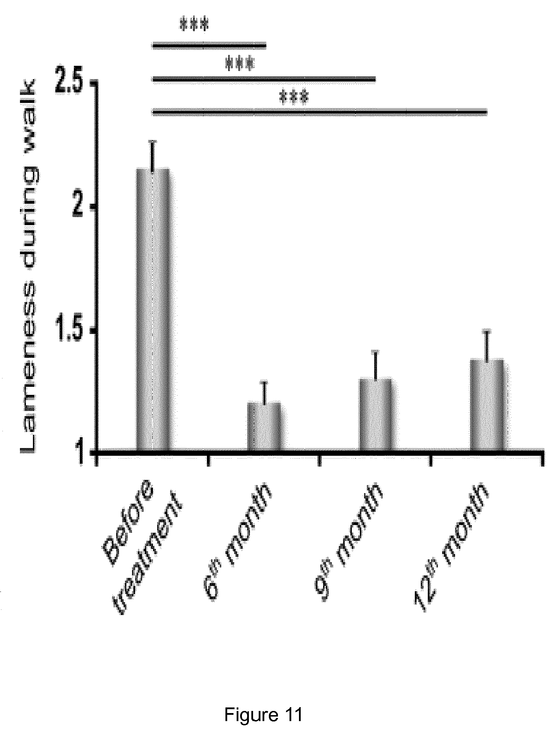

Figure 11 displays and summarizes the data of assessing the lameness of 40 dogs.

The data were evaluated statistically. Comparison between groups was done using non-parametric Friedman test and Tukey post hoc analysis.

### Example 8

### Characterization of the immunophenotype and differentiation of canine MSCs

### A: Flow cytometry

Passage 2 MSC cells were analysed for cell surface markers using FITC-conjugated rat antimouse/human CD44 (BioLegend, 1.2 ng/20 µl) and PE-conjugated rat anti-canine CD90 (eBioscience, 6 ng/ 20 µl) antibodies. Cells (10⁵ cells/sample) were washed with 1 ml IFB (immunofluorescence buffer: 1% FSC, 0.1% sodium-azide in PBS) and incubated with the antibodies for 30 minutes on ice. The cells were then washed with IFB and suspended in 250 µl IFB. The fluorescence of 10,000 cells per samples was measured with FACSCalibur (BD Biosciences). Evaluation of the data was carried out with the CellQuest software (BD Biosciences). The presence of CD45+ and CD34+ non-MSC cells was tested using FITC-conjugated rat anti-canine CD45 antibodies (eBioscience 10 ng/20 µl) and FITC-conjugated mouse anti-canine CD34 antibodies (R&D Systems 10 µl/sample), the same way as in the case of MSC-specific markers.

The experiment reveals that while the stromal vascular fraction (SVF) contains a significant amount of non-MSC cells, the culture contains only MSCs (CD44- and CD90- positive cells).

### B: Osteogenic differentiation

Passage 2 cells (5.1x10³/cm² in 24-well plates) were kept in osteogenic differentiation medium (10 mM β-glycerophosphate, 50 µg/ml L-ascorbic acid 2-phosphate and 10 nM dexamethasone (all from Sigma Aldrich) in DMEM/F12). The differentiation medium was changed every three days. On the 21st day, cells were washed with PBS and fixed with 8% formaldehyde for 15 min. After washing, staining was performed with 1% Alizarin Red S in distilled water for 45 min. Cells were analysed with an Olympus CKX41 microscope and photos were taken with an Olympus Digital Camera C-5060 from five non-overlapping regions at 100x magnification.

Calcium deposits with red staining indicate osteogenic differentiation in the treated cell cultures.

### C: Adipogenic differentiation

Adipogenic differentiation was stimulated by cultivating cells in DMEM/F12 medium containing 15% rabbit serum, 2mM Glutamine, and 50 U/ml Penicillin and Streptomycin. After 4 days, cells (in 24-well plates) were washed with PBS and fixed with 8% formaldehyde for 15 minutes. After washing, cells were stained with 1 ml/sample Adipo-Red solution (30 µl/ml AdipoRed™ (Lonza) and 1µg/ml Hoechst33342 in PBS) for 15 minutes. Samples were then washed twice with PBS and analysed with a fluorescent Olympus microscope (Olympus IX81) at 100x magnification.

Red staining in the cells reveals the accumulated fat droplets in the samples.

### D: Chondrogenic differentiation

Chondrogenic differentiation was induced in three-dimensional pellet culture according to Bosnakovski et al. [2]. Cells (1.7 x 10⁵ cells/200 µl DMEM/F12 15% FCS/well) were transferred to fifteen wells of a 96-well round bottom plate, centrifuged at 300xg for 5 minutes and cultured for 21 days changing the medium to fresh medium every 2-3 days.

After 21 days of culturing, samples were collected and digested with 0.3% collagenase (Sigma Aldrich) in Hank's buffer, which contained 2u/µl RNase inhibitor (RNasin Promega), for 30 minutes at 37°C. Total RNA was isolated using Perfect Pure RNA Kit (5Prime) according to the manufacturer's instructions. Samples without induction of three-dimensional differentiation (cell suspension not inducing differentiation) served as negative control. RevertAid H Minus First-Strand cDNA Synthesis Kit (Thermo Fisher Scientific, Inc.) was used to obtain cDNA. *Gapdh, Sox9,* and *Aggrecan* quantitative real-time PCR was performed on a RotoGene3000 instrument (Corbett Life Science) using AccuPower® 2X Greenstar qPCR Master Mix (Bioneer). Relative quantification of gene expression was performed by comparison of threshold values. All results were normalized to the expression of the housekeeping *Gapdh* gene. qPCR primers were used according to Lee et. al and Zang et al. with slight modifications [3],[4]. *Gapdh:* forward: TGGCAAAGTGGATATTGTCG reverse: AGATGGACTTCCCGTTGATG, *Sox9:* forward: ACGACTACACTGACCACCAGAAC reverse: GTAGGTGAAGGTGGAGTAGAGGC, *Aggrecan:* forward: TTGCACTCAGGAGAGGAGAC reverse: CCACGCAGGTGGCTCCATTC.

It is visible that three-dimensional culturing induced the expression of the cartilage-specific *Sox9* and *Aggrecan* genes.

### Arthroscopic examination

Arthroscopic records were taken from one dog before stem cell treatment and 12 months after transplantation. The dog was diagnosed with fissures of the medial coronoid process; therefore, subtotal coronoidectomy was carried out and the detached cartilage part was removed. Arthroscopic records taken before the stem cell transplantation and 12 months after clearly demonstrated that on the same site where the cartilage had been missing on the ulna, a significant amount of new cartilage tissue developed.

In the area of the missing cartilage (before transplantation, encircled area) new cartilage tissue developed.

### Histologic assessment of cartilage biopsies

Histologic analysis of cartilage biopsy specimens obtained from the regenerated area. After paraffin embedding and sectioning, the whole biopsy is shown stained with hematoxilin-eosin at 25x magnification Figure 15A. At 400x magnification, blue staining of a matrix containing glycoseaminoglycans is visible around the chondrocytes Figure 15B. Safranin-O staining demonstrates the presence of proteoglycans, which are typical components of the articular cartilage matrix Figure 15C. Different histologic stainings of the cartilage biopsy obtained from the regenerated area revealed the development of hyaline cartilage due to MSC treatment.

Biopsy specimens are obtained from the regenerated cartilage area identified by arthroscopy. Formalinfixed tissues were embedded in paraffin and 3-4 µm sections were cut. Sections were stained with hematoxilin-eosin (Figure 15A and B) or safranin-O (Figure 15C). Figures 15A and C show 25x magnification; Figure 15B shows 400x magnification.

### Example 9

### Changes in the level of lameness when treating joints other than the elbow joint

3 hock, 2 shoulder, 5 hip and 5 knee joints were treated during the programme in which follow-up was continued for 3 to 12 months. Improvement, that is, the decrease in lameness could be observed in all cases, and these improvements proved to be long lasting just as in the case of elbow joints.

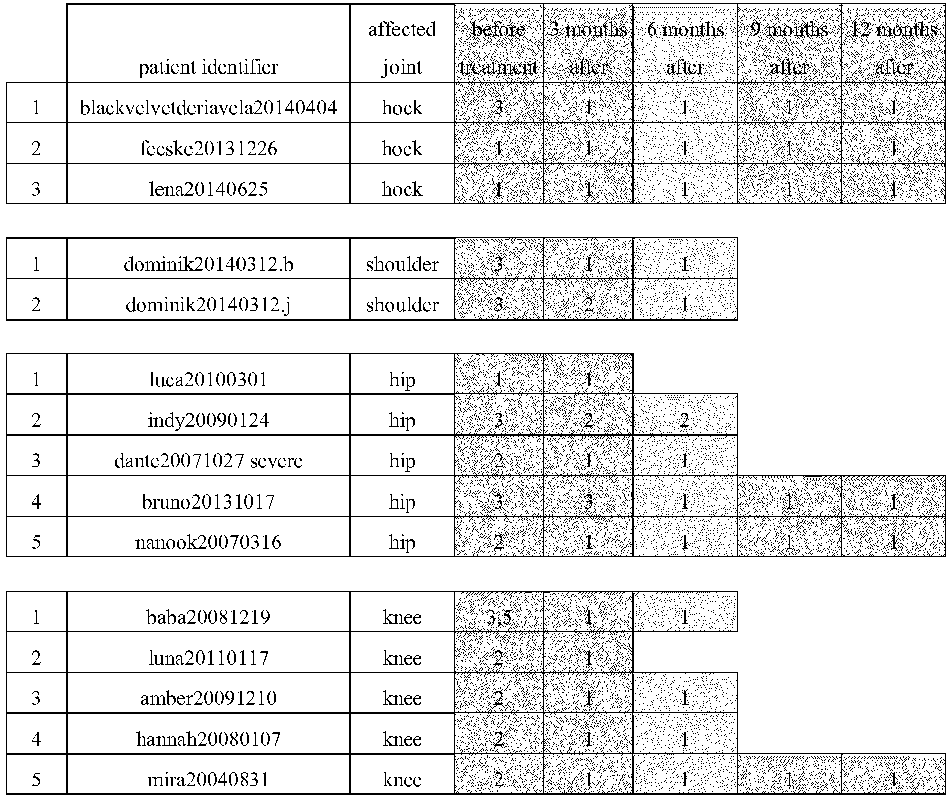

## Claims

1. Composition for the treatment of articular cartilage injury in a mammalian subject, wherein the composition comprises
- mesenchymal stem cells (MSC) obtainable by a method comprising isolating and culturing mesenchymal stem cells from at least two donor subjects of the mammalian species, wherein the donors are different from the subject to be treated
and
- from 0.1% to 1% by weight anionic nonsulfated glycosaminoglycan, preferably hyaluronic acid.

2. The composition according to claim 1 for the treatment of articular cartilage degeneration, preferably orthopaedic diseases with degenerative deformation of the cartilage, orthopaedic diseases with cartilage wear and/or cartilage detachment, osteoarthritis, dysplasia, traumas of the joint, for use within up to 72 hours, 48 hours or preferably within 24 hours of the preparation of the compound.

3. The composition according to claim 1 or 2, wherein the composition comprises mesenchymal stem cells (MSC) obtainable by a method comprising isolating and culturing mesenchymal stem cells from at least two subjects of the mammalian species different from the subject to be treated, wherein the cell culture is passaged at least once, preferably twice.

4. The composition according to any one of claims 1 to 3 for the treatment of a joint injury, preferably for the treatment of joint dysplasia, wherein the composition comprises MSCs derived from visceral adipose tissue of 2, 3 or 4 donor subjects, wherein the amounts of MSCs obtained from the visceral adipose tissue of each donor subject are essentially the same or differs from the same up to ±30%, preferably ±20% or ±10%.

5. The composition according to any one of claims 1 to 3, wherein
- the mammalian species is the dog and th
e composition comprises a total amount of 5 to 20 million MSCs isolated from two donor subjects different from the subject to be treated, in a donor subjects ratio of 0.4:0.6-0.6:0.4, in a suspension of 0.5-1.5 ml comprising from 0.2 to 0.8% hyaluronic acid and wherein the composition is formulated for use in administration into the joint, or
- wherein the mammalian species is the horse and
the composition comprises a total amount of 10 to 30 million MSCs isolated from two donor subjects different from the subject to be treated, in a donor subjects ratio of 0.4:0.6-0.6:0.4, in a suspension of 1-2.5 ml comprising from 0.2 to 0.8% hyaluronic acid and wherein the composition is formulated for use in administration into the joint.

6. A method for the preparation of a composition for the treatment of a joint injury, preferably joint dysplasia in a non-human mammalian patient, wherein
cultured mesenchymal stem cells (MSCs) are obtained by
(a) isolating portions of visceral adipose tissue from a plurality of subjects of the same species, wherein the subjects are different from the mammalian patient and then
(b) processing said portions by at least removing fat,
(c) obtaining said mesenchymal stem cells (MSCs) from said portions and culturing;
(d) portions of defined amounts of cultured cells obtained from said cultured MSCs are stored,
(f) the stored portions of MSCs are cultured prior to the preparation of the composition,
(g) a pool of cultured MSC portions is formed, wherein said pool comprises portions of MSCs obtained from the visceral adipose tissue of at least two healthy donor subjects of the mammalian species, wherein the donors are different from the subject to be treated,
(h) the pool of cultured MSCs is formulated into a composition by adding a biodegradable non-sulfated glycosaminglycan suitable to bind the MSCs and/or to maintain the viability of the MSCs and/or to stabilize the MSCs.

7. The method according to claim 6 for the production of a composition for the treatment of a joint injury, preferably joint dysplasia in a non-human mammalian patient, wherein before forming a pool or fraction of MSCs obtained from a plurality of subjects
a) portions of visceral adipose tissue are isolated from a plurality of subjects of the same species, wherein said subjects are different from the mammalian patient,
b) processed portions of adipose tissue containing cells are provided by processing the portions of visceral adipose tissue by removing fat,
c) mesenchymal stem cells (MSC) are obtained from the processed portions of adipose tissue, preferably by using collagenase, allowing the cells to sediment and taking up again, preferably re-suspending the cells,
d) MSCs are cultured by passaging the cells at least once, preferably twice or three times (but preferably not more than four times or not more than three times), preferably to at least 70%, 80% or preferably 90% confluency,
e) portions of defined amounts of cultured cells obtained from the individual portions of adipose tissue are brought separately from each other into long term storage form, preferably frozen.

8. The method according to claim 6 or 7 for the preparation of a composition for the treatment of a joint injury in a non-human mammalian patient,
wherein the cells are frozen,
before freezing the cells, culturing of said cells by at least one passage thereof is carried out,
and after thawing, said cells are cultured for no longer than 1-5 days, preferably for 2-4 days or for about 2 or 3 days,
then formulated into a composition (preferably by adding a non-sulfated glucosaminoglycan, preferably hyaluronic acid).

9. The method according to claim 6 or 7 for the preparation of a composition for the treatment of a joint injury in a non-human mammalian patient,
wherein culturing of said cells by at least one passage thereof is carried out after freezing the cells,
and then the cells are formulated into a composition (preferably by adding a non-sulfated glucosaminoglycan, preferably hyaluronic acid),
preferably the pool or fraction of MSCs obtained from a plurality of subjects is formed after thawing the frozen cultures, preferably as part of the formulation process of the composition.

10. The method of claim 6 for the preparation of a composition for the treatment of a joint injury in a non-human mammalian patient, wherein culturing of said cells by at least one passage thereof and forming the pool or fraction of MSCs obtained from a plurality of subjects are carried out prior to said freezing.

11. The method of claim 6 for the preparation of a composition for the treatment of a joint injury in a non-human mammalian patient,
wherein culturing of said cells by at least one passage thereof,
and then forming the pool or fraction of MSCs obtained from a plurality of subjects,
and formulation of the composition (preferably by adding a non-sulfated glycosaminoglycan, preferably hyaluronic acid) are carried out prior to said freezing, and
during formulation of the composition and after thawing, the cells are cultured up to 1-5 days, preferably for 2-4 or for about 2 days or for 3 days.

12. The method according to any one of claims 6-10, wherein the cells cultured in a culturing process comprising a passage are cultured for 2-20 days, preferably for 9-13 days.

13. The method according to any one of claims 6-10, wherein the pool comprises MSCs obtained from 2-10 adult mammalian subjects and the component present in the lowest ratio differs from the component present in the highest ratio, at most in 80% or in 50% or in 10%.

14. The method according to any one of claims 6-12, wherein the composition is for the treatment of joint dysplasia and wherein MSCs obtained from a plurality of donors are thawed for the treatment of a single joint affected by joint dysplasia such that the starting amount for the preparation of the composition for the treatment of a single joint is 0.1-20 million cells ( 1 x 10⁶ - 2 x 10⁷ cells) and the cells are cultured for 2-20 days, preferably for 9-13 days.

15. The method according to any one of claims 6-13, wherein the glycosaminoglycan is hyaluronic acid and the composition is formulated in an injectable form.


SEQUENCE LISTING

<110>  DeltaBio 2000 Kft.
       Magyar Tudományos Akadémia Szegedi Biológiai Kutatóközpont

<120>  METHOD AND PREPARATION FOR THE TREATMENT OF ORTHOPAEDIC
       DISEASES...

<130>  01659ep

<140>  EP16020175.2
<141>  2016-06-09

<150>  HU1500118
<151>  2015-05-08

<160>  6

<170>  PatentIn version 3.5

<210>  1
<211>  20
<212>  DNA
<213>  Artificial Sequence

<220>
<223>  Gapdh forward qPCR primer

<400>  1
tggcaaagtg gatattgtcg                                                  20


<210>  2
<211>  20
<212>  DNA
<213>  Artificial Sequence

<220>
<223>  Gapdh reverse qPCR primer

<400>  2
agatggactt cccgttgatg                                                  20


<210>  3
<211>  23
<212>  DNA
<213>  Artificial Sequence

<220>
<223>  Sox9 forward qPCR primer

<400>  3
acgactacac tgaccaccag aac                                              23


<210>  4
<211>  23
<212>  DNA
<213>  Artificial Sequence

<220>
<223>  Sox9 reverse qPCR primer

<400>  4
gtaggtgaag gtggagtaga ggc                                               23


<210>  5
<211>  20
<212>  DNA
<213>  Artificial Sequence

<220>
<223>  Aggrecan forward qPCR primer

<400>  5
ttgcactcag gagaggagac                                                   20


<210>  6
<211>  20
<212>  DNA
<213>  Artificial Sequence

<220>
<223>  Aggrecan reverse qPCR primer

<400>  6
ccacgcaggt ggctccattc                                                   20